(19) Europäisches Patentamt
European Patent Office
Office européen des brevets

(11) EP 2 683 751 B1

(12) **EUROPÄISCHE PATENTSCHRIFT**

(45) Veröffentlichungstag und Bekanntmachung des Hinweises auf die Patenterteilung:
**15.07.2015 Patentblatt 2015/29**

(21) Anmeldenummer: **12712578.9**

(22) Anmeldetag: **03.03.2012**

(51) Int Cl.:
***C08F 220/58*** *(2006.01)*

(86) Internationale Anmeldenummer:
**PCT/EP2012/000968**

(87) Internationale Veröffentlichungsnummer:
**WO 2012/119746 (13.09.2012 Gazette 2012/37)**

(54) **POLYMERE AUF BASIS VON SULFONSÄUREN, AMIDEN UND SPEZIELLEN VERNETZERN**

POLYMERS BASED ON SULFONIC ACIDS, AMIDES AND SPECIAL CROSS-LINKING AGENTS

POLYMÈRES À BASE D’ACIDES SULFONIQUES, D’AMIDES ET D’AGENTS DE RÉTICULATION SPÉCIFIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.03.2011 DE 102011013341**

(43) Veröffentlichungstag der Anmeldung:
**15.01.2014 Patentblatt 2014/03**

(73) Patentinhaber: **Clariant Finance (BVI) Limited**
**Road Town, Tortola (VG)**

(72) Erfinder:
- **KLUG, Peter**
**63762 Großostheim (DE)**
- **FISCHER, Dirk**
**55270 Klein-Winterheim (DE)**
- **LINDNER, Thomas**
**65197 Wiesbaden (DE)**
- **MÜCKENHEIM, Wiebke**
**65812 Bad Soden (DE)**
- **BRASCH, Bianca**
**97773 Aura im Sinngrund (DE)**
- **HORNUNG, Michael**
**65929 Frankfurt am Main (DE)**

(74) Vertreter: **Paczkowski, Marcus**
**Clariant Produkte (Deutschland) GmbH**
**Patent Management**
**Industriepark Höchst, G 860**
**65926 Frankfurt am Main (DE)**

(56) Entgegenhaltungen:
**DE-A1-102009 014 877** **FR-A1- 2 910 899**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 683 751 B1

## Beschreibung

Die vorliegende Erfindung betrifft Polymere auf Basis von speziellen Monomeren mit Sulfonsäuregruppen oder den entsprechenden Sulfonaten wie z. B. 2-Acrylamido-2-methyl-propansulfonsäure oder ihren Salzen, speziellen offenkettigen Acryl-, Methacryl- oder Ethacrylamiden und speziellen trifunktionellen Vernetzern, ein Verfahren zur Herstellung derartiger Polymere, kosmetische, dermatologische oder pharmazeutische Zusammensetzungen enthaltend ein oder mehrere derartige Polymere sowie bestimmte Verwendungen derartiger Polymere wie z. B. die Verwendung derartiger Polymere als Sensorikadditiv, Verdicker oder Konsistenzgeber.

Polymere auf Basis von 2-Acrylamido-2-methyl-propansulfonsäure haben sich als vielseitige Gelbildner und Verdicker in kosmetischen, dermatologischen und pharmazeutischen Zusammensetzungen über die letzten Jahre im Markt etabliert.

Beispielsweise beschreibt die EP 0 816 403 (Clariant) wasserlösliche oder wasserquellbare Polymere auf Basis von 2-Acrylamido-2-methylpropansulfonaten. Als vernetzende Monomere werden Dipropylenglykoldiallylether, Polyglykoldiallylether, Triethylenglykoldivinylether, Hydrochinondiallylether, Tetraallyloxyethan, Tetraethylenglykoldiacrylat, Triallylamin, Trimethylolpropandiallylether, Methylenbisacrylamid, Divinylbenzol, Trimethylolpropantriacrylat (TMPTA) und Trimethylolpropantrimethacrylat (TMPTMA) genannt.

Diese Polymere quellen in Wasser über einen weiten pH-Bereich und sind einfach zu handhaben. Sie haben in der kosmetischen Industrie ein weites Anwendungsfeld gefunden, wie beispielsweise in EP 0 815 843, EP 0 815 844, EP 0 815 845, EP 0 815 846, EP 0 815 847, EP 0 815 828, EP 0 829 258, EP 1 136 058, EP 1 325 729 und EP 1 468 670 (L'Oréal) beschrieben.

WO 2010/108634 (Clariant) beschreibt beispielsweise vernetzte Polymere enthaltend Struktureinheiten abgeleitet von der 2-Acrylamido-2-methylpropansulfonsäure bzw. von ihren Salzen und Carboxyethylacrylat. Die Polymere eignen sich speziell als Verdicker und Stabilisatoren durch den Aufbau einer Fließgrenze in tensidhaltigen Zusammensetzungen.

EP 1 746 114 (Shiseido) beschreibt z. B. vernetzte Polymere aus Struktureinheiten abgeleitet von der 2-Acrylamido-2-methyl-propansulfonsäure bzw. von ihren Salzen und Hydroxyethylacrylamid. Als vernetzende Monomere werden Ethylenglykoldiacrylat, Ethylenglykoldimethacrylat, Polyoxyethylendiacrylat, Polyoxyethylendimethacrylat, Diethylenglykoldimethacrylat, Trimethylolpropantriacrylat, N,N-Methylen-bis-acrylamid, N,N-Ethylen-bis-acrylamid, Triallylisocyanurat und Pentaerythritdimethacrylat genannt.

WO 2010/009953 (Henkel) beschreibt beispielsweise Stylingmittel enthaltend vernetzte Polymere aus 2-Acrylamido-2-methyl-propansulfonsäure bzw. ihren Salzen, Dimethylacrylamid und Acrylsäure.

EP 1 116 733 (Clariant) beschreibt z. B. vernetzte Polymere enthaltend Struktureinheiten hervorgegangen aus 2-Acrylamido-2-methyl-propansulfonsäure bzw. ihren Salzen und Vinylamiden. Die Polymere weisen ein exzellentes, frisches Hautgefühl und eine geringe Klebrigkeit auf. Als vernetzende Monomere werden Allylacrylat, Allylmethacrylat, Dipropylenglykoldiallylether, Polyglykoldiallylether, Triethylenglykoldivinylether, Hydrochinondiallylether, Tetraallyloxyethan, Tetraethylenglykoldiacrylat, Triallylamin, Trimethylolpropandiallylether, N,N-Methylen-bis-acrylamid, Divinylbenzol und Trimethylolpropantriacrylat genannt. Die beschriebenen Polymere sind jedoch in ihrer langfristigen Pflegewirkung verbesserungsbedürftig.

Vernetzte Polymere aus 2-Acrylamido-2-methyl-propansulfonsäure bzw. ihren Salzen und einem Dialkylacrylamid sind aus JP 2005/206607 (Shiseido) bekannt. Als vernetzende Monomere werden Ethylenglykoldiacrylat, Ethylenglykoldimethacrylat, Polyoxyethylendiacrylat, Polyoxyethylendimethacrylat, Diethylenglykoldimethacrylat, Trimethylolpropantriacrylat, N,N-Methylen-bis-acrylamid, N,N-Ethylen-bis-acrylamid, Triallylisocyanurat und Pentaerythritdimethacrylat genannt.

Mit den oben genannten vernetzten Polymeren sind jedoch nicht alle sensorischen Eigenschaftsprofile in kosmetischen, dermatologischen oder pharmazeutischen Zusammensetzungen abdeckbar und zudem ist ihre Verdickungsleistung in Gegenwart von Salz oftmals verbesserungsbedürftig.

Aufgabe der vorliegenden Erfindung war es daher, Polymere bereitzustellen, die insbesondere in kosmetischen, dermatologischen und pharmazeutischen Zusammensetzungen ein sensorisch optimiertes Eigenschaftsprofil, wie z. B. ein reichhaltiges, pflegendes Hautgefühl liefern und zudem in vorteilhafter Weise als Verdicker auch in salzhaltigen Zusammensetzungen wie in Wasser enthaltenden salzhaltigen Zusammensetzungen geeignet sind.

Überraschend wurde gefunden, dass diese Aufgabe mit Polymeren auf Basis von speziellen Monomeren mit Sulfonsäuregruppen oder ihren Salzen der nachfolgenden Formel (1) wie z. B. 2-Acrylamido-2-methyl-propansulfonsäure oder -sulfonaten, speziellen offenkettigen Amiden der nachfolgenden Formel (2) und speziellen trifunktionellen Vernetzern der nachfolgenden Formel (5) gelöst wird.

Gegenstand der Erfindung sind daher wasserlösliche oder wasserquellbare Polymere enthaltend:

a) 20,0 bis 98,97 mol-%, vorzugsweise 40,0 bis 97,7 mol-% und besonders bevorzugt 60,0 bis 93,75 mol-% an einer oder mehreren voneinander unabhängigen wiederkehrenden Struktureinheiten der Formel (1)

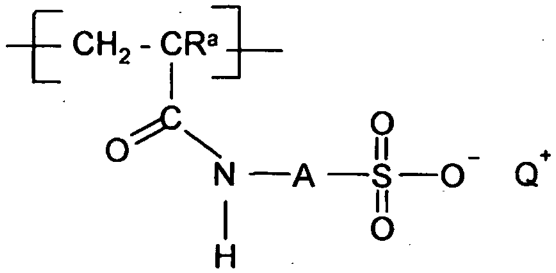

(1)

worin

- $R^a$ Wasserstoff, Methyl oder Ethyl bedeutet,
- A lineares oder verzweigtes $C_1$-$C_{12}$-Alkylen und vorzugsweise lineares oder verzweigtes $C_1$-$C_8$-Alkylen bedeutet, und
- $Q^+$ für ein Gegenion steht,

und

b) 1,0 bis 60,0 mol-%, vorzugsweise 2,0 bis 39,9 mol-% und besonders bevorzugt 5,0 bis 29,5 mol-% an einer oder mehreren voneinander unabhängigen wiederkehrenden Struktureinheiten der Formel (2)

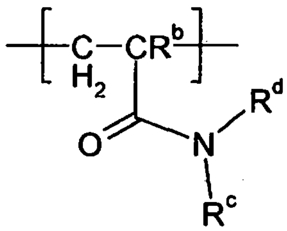

(2)

worin

- $R^b$ Wasserstoff, Methyl oder Ethyl bedeutet,
- $R^c$ Wasserstoff, eine lineare oder verzweigte Alkylgruppe mit 1 bis 50, vorzugsweise 1 bis 10 und besonders bevorzugt 1 bis 6 Kohlenstoffatomen, eine lineare oder verzweigte Mono-Hydroxyalkylgruppe mit 2 bis 6 und vorzugsweise 2 bis 5 Kohlenstoffatomen oder eine lineare oder verzweigte Di-Hydroxyalkylgruppe mit 2 bis 6 und vorzugsweise 2 bis 5 Kohlenstoffatomen bedeutet und
- $R^d$ eine lineare oder verzweigte Alkylgruppe mit 1 bis 50, vorzugsweise 1 bis 10 und besonders bevorzugt 1 bis 6 Kohlenstoffatomen, eine lineare oder verzweigte Mono-Hydroxyalkylgruppe mit 2 bis 6 und vorzugsweise 2 bis 5 Kohlenstoffatomen oder eine lineare oder verzweigte Di-Hydroxyalkylgruppe mit 2 bis 6 und vorzugsweise 2 bis 5 Kohlenstoffatomen bedeutet,

und

d) 0,01 bis 8,0 mol%, vorzugsweise 0,1 bis 5,0 mol-% und besonders bevorzugt 0,25 bis 3,0 mol-% an einer oder mehreren voneinander unabhängigen wiederkehrenden vernetzenden Struktureinheiten der Formel (5)

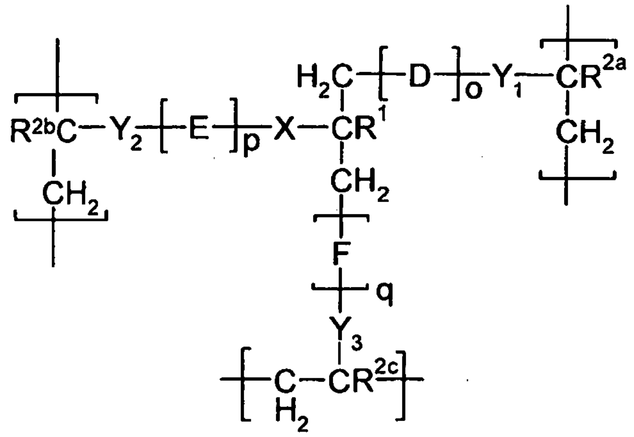

(5)

worin

| | |
|---|---|
| $R^1$ | Wasserstoff, Methyl, Ethyl, Methylol ($-CH_2-OH$) oder Ethylol ($-CH_2-CH_2-OH$) bedeutet, |
| $R^{2a}$, $R^{2b}$ und $R^{2c}$ | jeweils unabhängig voneinander Wasserstoff, Methyl oder Ethyl bedeuten, |
| X | eine chemische Bindung, Methylen ($-CH_2-$), Ethylen ($-CH_2-CH_2-$) oder eine lineare oder verzweigte Alkylengruppe mit 3 Kohlenstoffatomen bedeutet, |
| $Y_1$, $Y_2$ und $Y_3$ | jeweils unabhängig voneinander eine chemische Bindung, O, $CH_2$, C(O)O, OC(O), $C(O)NR^3$ oder $NR^3C(O)$ bedeuten, |
| $R^3$ | Wasserstoff oder einen linearen oder verzweigten Alkylrest mit 1 bis 50 C-Atomen bedeutet, |
| D, E und F | jeweils unabhängig voneinander Methylenoxy, Ethylenoxy, Propylenoxy, eine lineare oder verzweigte Alkylengruppe mit 1 bis 6 Kohlenstoffatomen, eine lineare oder verzweigte, einfach oder mehrfach ungesättigte Alkenylengruppe mit 2 bis 6 Kohlenstoffatomen, eine lineare oder verzweigte Mono-Hydroxyalkylengruppe mit 2 bis 6 Kohlenstoffatomen oder eine lineare oder verzweigte Di-Hydroxyalkylengruppe mit 3 bis 6 Kohlenstoffatomen bedeuten, |
| o, p und q | jeweils unabhängig voneinander ganze Zahlen von 0 bis 50 bedeuten, und die Summe $o + p + q \geq 3$ ist. |

In den Definitionen D, E und F der Struktureinheiten der Formel (5) bedeutet "Methylenoxy" $-CH_2-O-$ oder $-O-CH_2-$, "Ethylenoxy" $-CH_2-CH_2-O-$ oder $-O-CH_2-CH_2-$ und "Propylenoxy" $-CH(CH_3)-CH_2-O-$, $-CH_2-CH(CH_3)-O-$, $-O-CH(CH_3)-CH_2-$ oder $-O-CH_2-CH(CH_3)-$.

Die im Rahmen der vorliegenden Erfindung angegebenen Mengen (in mol-%) für die Struktureinheiten der Formeln (1), (2) und (5) [sowie für die später beschriebenen Struktureinheiten der Formeln (3) und (4)] beziehen sich auf die Gesamtmenge aller in den erfindungsgemäßen Polymeren enthaltenen wiederkehrenden Struktureinheiten.

In einem erfindunsgemäßen Polymer können jeweils verschiedene Struktureinheiten der Formel (1) und/oder der Formel (2) und/oder der Formel (5) enthalten sein.

Die erfindungsgemäßen Polymere sind u. a. hervorragend geeignet als Verdicker von wässrigen Systemen und als Konsistenzgeber, insbesondere in kosmetischen, dermatologischen oder pharmazeutischen Zusammensetzungen. Sie zeigen im Vergleich zu Polymeren aus dem Stand der Technik insbesondere ein pflegenderes Hautgefühl. Darüber hinaus bewirken sie auch eine höhere Viskosität, insbesondere in salzhaltigen Zusammensetzungen wie z. B. in Wasser enthaltenden salzhaltigen Zusammensetzungen. Zudem zeigen sie eine vorteilhafte Salzstabilität. Außerdem weisen sie auch eine vorteilhafte langfristige Pflegewirkung auf. Vorteilhafterweise zeigen sie zudem über einen weiten pH-Bereich, d. h. auch bei stark sauren pH-Werten, gute Verdickungseigenschaften.

In der einen oder den mehreren Struktureinheiten der Formel (1) der erfindungsgemäßen Polymere ist $R^a$ vorzugsweise Wasserstoff oder Methyl und besonders bevorzugt Wasserstoff.

In der einen oder den mehreren Struktureinheiten der Formel (1) der erfindungsgemäßen Polymere ist A vorzugsweise eine Struktureinheit der Formel $-C(CH_3)_2-CH_2-$.

Besonders bevorzugt ist die eine oder sind die mehreren Struktureinheiten der Formel (1) der erfindungsgemäßen Polymere abgeleitet von der 2-Acrylamido-2-methyl-propansulfonsäure oder ihren Salzen.

Wie bereits erwähnt können in einem erfindunsgemäßen Polymer beispielsweise verschiedene Struktureinheiten der Formel (1) enthalten sein. Ein erfindungsgemäßes Polymer kann beispielsweise mehrere Struktureinheiten der Formel (1) enthalten, die sich durch unterschiedliche Gegenionen $Q^+$ voneinander unterscheiden.

In der einen oder den mehreren Struktureinheiten der Formel (1) der erfindungsgemäßen Polymere steht das Gegenion $Q^+$ vorzugsweise für $H^+$, $NH_4^+$, organische Ammoniumionen $[HNR^5R^6R^7]^+$, wobei $R^5$, $R^6$ und $R^7$ unabhängig

voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste $R^5$, $R^6$ und $R^7$ nicht Wasserstoff ist, $Alkali^+$, wobei unter $Alkali^+$ wiederum $Li^+$, $Na^+$ und $K^+$ bevorzugt sind, ½ $Erdalkali^{++}$, wobei unter ½ $Erdalkali^{++}$ wiederum ½ $Ca^{++}$ und ½ $Mg^{++}$ bevorzugt sind, ½ $Zn^{++}$ oder ⅓ $Al^{+++}$ oder für Mischungen aus diesen Ionen.

In der einen oder den mehreren Struktureinheiten der Formel (1) der erfindungsgemäßen Polymere steht das Gegenion $Q^+$ besonders bevorzugt für $H^+$, $NH_4^+$, $Alkali^+$, wobei unter $Alkali^+$ wiederum $Na^+$ bevorzugt ist, ½ $Erdalkali^{++}$, wobei unter ½ $Erdalkali^{++}$ wiederum ½ $Ca^{++}$ und ½ $Mg^{++}$ bevorzugt sind, oder für Mischungen aus diesen Ionen. Insbesondere bevorzugt ist das Gegenion $Q^+$ in der einen oder den mehreren Struktureinheiten der Formel (1) der erfindungsgemäßen Polymere ausgewählt aus der Gruppe bestehend aus $H^+$, $NH_4^+$ und $Na^+$ und darunter bevorzugt ist $Q^+$ wiederum ausgewählt aus der Gruppe bestehend aus $H^+$ und $NH_4^+$.

Vorzugsweise ist der Neutralisationsgrad der einen oder der mehreren Struktureinheiten der Formel (1) der erfindungsgemäßen Polymere von 50,0 bis 100 mol-%, besonders bevorzugt von 80,0 bis 100 mol%, insbesondere bevorzugt von 90,0 bis 100 mol-% und außerordentlich bevorzugt von 95,0 bis 100 mol%.

In der einen oder den mehreren Struktureinheiten der Formel (2) der erfindungsgemäßen Polymere ist $R^b$ vorzugsweise Wasserstoff oder Methyl.

In einer bevorzugten Ausführungsform der Erfindung ist in der einen oder den mehreren Struktureinheiten der Formel (2) der erfindungsgemäßen Polymere $R^b$ Wasserstoff oder Methyl und vorzugsweise Wasserstoff, $R^c$ Wasserstoff, eine lineare oder verzweigte Alkylgruppe mit 1 bis 6 Kohlenstoffatomen oder eine lineare oder verzweigte Mono-Hydroxyalkylgruppe mit 2 bis 6 Kohlenstoffatomen und $R^d$ eine lineare oder verzweigte Alkylgruppe mit 1 bis 6 Kohlenstoffatomen oder eine lineare oder verzweigte Mono-Hydroxyalkylgruppe mit 2 bis 6 Kohlenstoffatomen.

In einer besonders bevorzugten Ausführungsform der Efindung ist in der einen oder den mehreren Struktureinheiten der Formel (2) der erfindungsgemäßen Polymere $R^b$ Wasserstoff oder Methyl und vorzugsweise Wasserstoff, $R^c$ Wasserstoff oder eine lineare oder verzweigte Alkylgruppe mit 1 bis 6 und vorzugsweise 1 bis 5 Kohlenstoffatomen und $R^d$ eine lineare oder verzweigte Alkylgruppe mit 1 bis 6 und vorzugsweise 1 bis 5 Kohlenstoffatomen oder eine lineare oder verzweigte Mono-Hydroxyalkylgruppe mit 2 bis 6 und vorzugsweise 2 bis 5 Kohlenstoffatomen.

In einer insbesondere bevorzugten Ausführungsform der Erfindung ist in der einen oder sind in den mehreren Struktureinheiten der Formel (2) $R^b$ Wasserstoff oder Methyl und vorzugsweise Wasserstoff, $R^c$ Wasserstoff, Methyl, Ethyl, n-Propyl oder iso-Propyl und $R^d$ Methyl, Ethyl, n-Propyl, iso-Propyl oder Hydroxyethyl.

Außerordentlich bevorzugt ist die eine oder sind die mehreren Struktureinheiten der Formel (2) der erfindungsgemäßen Polymere abgeleitet von einer oder mehreren Verbindungen ausgewählt aus der Gruppe bestehend aus Dimethylacrylamid, Hydroxethylacrylamid und Isopropylacrylamid.

In einer außerordentlich bevorzugten Ausführungsform der Erfindung enthalten die erfindungsgemäßen Polymere Struktureinheiten der Formel (2) abgeleitet von Dimethylacrylamid. Sofern die erfindungsgemäßen Polymere derartige Struktureinheiten enthalten, sind sie in einer darunter bevorzugten Ausführungsform der Erfindung in einer Menge von 10,0 bis 25 mol-% in den erfindungsgemäßen Polymeren enthalten.

In einer weiteren außerordentlich bevorzugten Ausführungsform der Erfindung enthalten die erfindungsgemäßen Polymere Struktureinheiten der Formel (2) abgeleitet von Hydroxethylacrylamid. Sofern die erfindungsgemäßen Polymere derartige Struktureinheiten enthalten, sind sie in einer darunter bevorzugten Ausführungsform der Erfindung in einer Menge von 10,0 bis 25 mol-% in den erfindungsgemäßen Polymeren enthalten.

In einer weiteren außerordentlich bevorzugten Ausführungsform der Erfindung enthalten die erfindungsgemäßen Polymere Struktureinheiten der Formel (2) abgeleitet von Isopropylacrylamid. Sofern die erfindungsgemäßen Polymere derartige Struktureinheiten enthalten, sind sie in einer darunter bevorzugten Ausführungsform der Erfindung in einer Menge von 10,0 bis 25 mol-% in den erfindungsgemäßen Polymeren enthalten.

In einer weiteren außerordentlich bevorzugten Ausführungsform der Erfindung enthalten die erfindungsgemäßen Polymere Mischungen aus Struktureinheiten der Formel (2) abgeleitet von Dimethylacrylamid und Hydroxyethylacrylamid. Sofern die erfindungsgemäßen Polymere Mischungen aus derartigen Struktureinheiten enthalten, sind diese Mischungen in einer darunter bevorzugten Ausführungsform der Erfindung in einer Menge von 10,0 bis 25 mol-% in den erfindungsgemäßen Polymeren enthalten.

In der einen oder den mehreren Struktureinheiten der Formel (5) der erfindungsgemäßen Polymere ist $R^1$ vorzugsweise Wasserstoff, Ethyl oder Methylol und besonders bevorzugt Wasserstoff oder Ethyl.

In der einen oder den mehreren Struktureinheiten der Formel (5) der erfindungsgemäßen Polymere sind $R^{2a}$, $R^{2b}$ und $R^{2c}$ jeweils unabhängig voneinander vorzugsweise Wasserstoff oder Methyl und besonders bevorzugt Wasserstoff.

In der einen oder den mehreren Struktureinheiten der Formel (5) der erfindungsgemäßen Polymere ist X vorzugsweise eine chemische Bindung, Methylen, oder Ethylen und besonders bevorzugt eine chemische Bindung oder Methylen.

In der einen oder den mehreren Struktureinheiten der Formel (5) bedeuten $Y_1$, $Y_2$ und $Y_3$ jeweils unabhängig voneinander vorzugsweise C(O)O, OC(O), C(O)$NR^3$ oder $NR^3$C(O) und besonders bevorzugt C(O)O oder OC(O).

$R^3$ ist vorzugsweise Wasserstoff oder ein linearer oder verzweigter Alkylrest mit 1 bis 10 und vorzugsweise 1 bis 6 C-Atomen.

In der einen oder den mehreren Struktureinheiten der Formel (5) der erfindungsgemäßen Polymere sind D, E und F jeweils unabhängig voneinander vorzugsweise Methylenoxy, Ethylenoxy oder Propylenoxy und besonders bevorzugt Ethylenoxy oder Propylenoxy.

Wie bereits erwähnt kann ein erfindungsgemäßes Polymer beispielsweise mehrere Struktureinheiten der Formel (5) enthalten. Diese mehreren Struktureinheiten der Formel (5) können sich beispielsweise durch den Grad der Ethoxylierung und/oder Propoxylierung voneinander unterscheiden.

Sofern in den Struktureinheiten $-[-D-]_o-$, $-[-E-]_p-$ und $-[-F-]_q-$ der Formel (5) eine oder mehrere der Laufzahlen o, p oder q eine ganze Zahl > 1 ist, können die jeweiligen Struktureinheiten aus einer einzigen oder auch aus unterschiedlichen Einheiten aufgebaut sein. Ist beispielsweise die Lauzahl "o" = 3, dann kann die Struktureinheit $-[-D-]_o-$ z. B. aus 3 Ethylenoxy-Einheiten aufgebaut sein. Sie kann dann aber z. B. auch aus 2 Ethylenoxy-Einheiten und 1 Propylenoxy-Einheit aufgebaut sein (oder umgekehrt) oder sie kann dann z. B. auch aus 1 Methylenoxy-Einheit, 1 Ethylenoxy-Einheit und 1 Propylenoxy-Einheit aufgebaut sein, u.s.w. Auch alle anderen Kombinationen aus Einheiten innerhalb der Struktureinheiten $-[-D-]_o-$, $-[-E-]_p-$ und $-[-F-]_q-$ sind möglich, solange die Definition der Gruppen D, E und F und der Laufzahlen o, p und q beachtet wird. Innerhalb der Struktureinheiten $-[-D-]_o-$, $-[-E-]_p-$ und $-[-F-]_q-$ kann die Verteilung der einzelnen Einheiten statistisch, alternierend, gradientenartig oder blockartig sein und ist vorzugsweise statistisch oder blockartig.

In der einen oder den mehreren Struktureinheiten der Formel (5) der erfindungsgemäßen Polymere können die einzelnen Struktureinheiten $-[-D-]_o-$, $-[-E-]_p-$ und $-[-F-]_q-$ wie soeben beschrieben aus unterschiedlichen Einheiten aufgebaut sein. In diesem Fall sind die entsprechenden Einheiten vorzugsweise ausgewählt aus der Gruppe bestehend aus Methylenoxy, Ethylenoxy und Propylenoxy und besonders bevorzugt ausgewählt aus der Gruppe bestehend aus Ethylenoxy und Propylenoxy.

In der einen oder den mehreren Struktureinheiten der Formel (5) der erfindungsgemäßen Polymere sind o, p und q jeweils unabhängig voneinander vorzugsweise ganze Zahlen von 0 bis 30, besonders bevorzugt ganze Zahlen von 0 bis 15 und insbesondere bevorzugt ganze Zahlen von 0 bis 10

In der einen oder den mehreren Struktureinheiten der Formel (5) der erfindungsgemäßen Polymere ist die Summe o + p + q vorzugsweise von 3 bis 90, besonders bevorzugt von 3 bis 45, insbesondere bevorzugt von 3 bis 30 und außerordentlich bevorzugt von 3 bis 20.

In einer bevorzugten Ausführungsform der Erfindung gehen die eine oder die mehreren vernetzenden Struktureinheiten der Formel (5) der erfindungsgemäßen Polymere vorzugsweise hervor aus ethoxylierten tri-Acryl- oder ethoxylierten tri-Methacrylsäureestern, ethoxylierten tri-Acryl- oder ethoxylierten tri-Methacrylsäureamiden, propoxylierten tri-Acryl- oder propoxylierten tri-Methacrylsäureestern, propoxylierten tri-Acryl- oder propoxylierten tri-Methacrylsäureamiden, statistisch oder blockartig funktionalisierten ethoxylierten/propoxylierten tri-Acryl- oder statistisch oder blockartig funktionalisierten ethoxylierten/propoxylierten tri-Methacrylsäureestern, ethoxylierten/propoxylierten tri-Acryl- oder ethoxylierten/propoxylierten tri-Methacrylsäureamiden, oder anderen ethoxylierten tri-Acryl- oder ethoxylierten tri-Methacrylsäureestern, ethoxylierten tri-Acryl- oder ethoxylierten tri-Methacrylsäureamiden multifunktioneller Alkohole, oder anderen propoxylierten tri-Acryl- oder propoxylierten tri-Methacrylsäureestern, propoxylierten tri-Acryl- oder propoxylierten tri-Methacrylsäureamiden multifunktioneller Alkohole, oder anderen statistisch oder blockartig funktionalisierten ethoxylierten/propoxylierten tri-Acryl- oder ethoxylierten/propoxylierten tri-Methacrylsäureestern, ethoxylierten/propoxylierten tri-Acryl- oder ethoxylierten/propoxylierten tri-Methacrylsäureamiden anderer multifunktioneller Alkohole,

oder ethoxylierten Glycerintriacrylaten oder -methacrylaten, oder propoxylierten Glycerintriacrylaten oder -methacrylaten oder anderen statistisch oder blockartig funktionalisierten ethoxylierten/propoxylierten Glycerintriacrylaten, oder -methacrylaten oder ethoxylierten Glycerintriacryamiden oder -methacrylamiden, oder propoxylierten Glycerintriacryamiden oder -methacrylamiden oder anderen statistisch oder blockartig funktionalisierten ethoxylierten/propoxylierten Glyceritriacrylamiden, oder -methacrylamiden,

oder ethoxylierten Trimethylolpropantriacrylaten oder -trimethacrylaten oder propoxylierten Trimethylolpropantriacrylaten oder -trimethacrylaten oder anderen statistisch oder blockartig funktionalisierten ethoxylierten/propoxylierten Trimethylolpropantriacrylaten oder -trimethacrylaten oder ethoxylierten Trimethylolpropantriacrylamiden oder -trimethacrylamiden oder propoxylierten Trimethylolpropantriacrylamiden oder -trimethacrylamiden oder anderen statistisch oder blockartig funktionalisierten ethoxylierten/propoxylierten Trimethylolpropantriacrylamiden oder -trimethacrylamiden,

oder ethoxylierten Pentaerythritoltriacrylaten oder -trimethacrylaten oder propoxylierten Pentaerythritoltriacrylaten oder -trimethacrylaten oder anderen statistisch oder blockartig funktionalisierten ethoxylierten/propoxylierten Pentaerythritoltriacrylaten oder -trimethacrylaten oder ethoxylierten Pentaerythritoltriacrylamiden oder -trimethacrylamiden oder propoxylierten Pentaerythritoltriacrylamiden oder -trimethacrylamiden oder anderen statistisch oder blockartig funktionalisierten ethoxylierten/propoxylierten Pentaerythritoltriacrylamiden oder -trimethacrylamiden.

In einer besonders bevorzugten Ausführungsform der Erfindung ist in der einen oder den mehreren Struktureinheiten der Formel (5) $R^1$ Wasserstoff oder Ethyl, $R^{2a}$, $R^{2b}$ und $R^{2c}$ sind jeweils unabhängig voneinander Wasserstoff oder Methyl, X ist eine chemische Bindung oder Methylen, $Y_1$, $Y_2$ und $Y_3$ sind jeweils unabhängig voneinander C(O)O oder OC(O), D, E und F sind jeweils unabhängig voneinander Ethylenoxy oder Propylenoxy, o, p und q sind jeweils unabhängig voneinander ganze Zahlen von 0 bis 30 und die Summe o + p + q ist von 3 bis 20.

Hierunter wiederum bevorzugt sind die erfindungsgemäßen Polymere auf Basis von Glycerin oder auf Basis von Trimethylolpropan aufgebaut.

Besonders bevorzugt als Vernetzer für die erfindungsgemäßen Polymere, d. h. als Verbindungen, aus denen die eine oder die mehreren Struktureinheiten der Formel (5) hervorgehen, sind
Glycerinpropoxylattriacrylat (3/3 PO/OH; PO = Propylenoxy; es wurden bei der Synthese 3 Mol Propylenoxid für 3 Mol OH-Gruppen des Glycerins verwendet) (GPTA),
Glycerinethoxylattriacrylat (3/3 EO/OH; EO = Ethylenoxy) (GETA),
Glycerinpropoxylattriacrylat (15/3 PO/OH) ($GP_{15}TA$),
Glycerinethoxylattriacrylat (15/3 EO/OH) ($GE_{15}TA$),
Glycerinpropoxyethoxylattriacrylat (15/3 PO-EO/OH) (GPETA),
Trimethylolpropanpropoxytriacrylat (3/3 PO/OH) (TMPTA-PO-3),
Trimethylolpropanethoxytriacrylat (3/3 EO/OH) (TMPTA-EO-3),
Trimethylolpropanethoxytriacrylat (6/3 EO/OH) (TMPTA-EO-6),
Trimethylolpropanethoxytriacrylat (15/3 EO/OH) (TMPTA-EO-15) und
Trimethylolpropanpropoxylethoxytriacrylat (15/3 PO-EO/OH) (TMPTA-PO-EO-15).

Insbesondere bevorzugt sind
Glycerinpropoxylattriacrylat (GPTA),
Glycerinpropoxylattriacrylat (15/3 PO/OH) ($GP_{15}TA$),
Trimethylolpropanpropoxytriacrylat (3/3 PO/OH) (TMPTA-PO-3),
Trimethylolpropanethoxytriacrylat (3/3 EO/OH) (TMPTA-EO-3) und
Trimethylolpropanethoxytriacrylat (15/3 EO/OH) (TMPTA-EO-15).

Außerordentlich bevorzugt sind
Glycerinpropoxylattriacrylat (GPTA),
Trimethylolpropanpropoxytriacrylat (3/3 PO/OH) (TMPTA-PO-3) und
Trimethylolpropanethoxytriacrylat (3/3 EO/OH) (TMPTA-EO-3).

Unter diesen Verbindungen ist wiederum Glycerinpropoxylattriacrylat (GPTA) bevorzugt.

Weiterhin bevorzugte Mengen für die eine oder die mehreren Struktureinheiten der Formel (5) in den erfindungsgemäßen Polymeren, insbesondere für Struktureinheiten der Formel (5), die aus den soeben explizit genannten Vernetzern hervorgehen, sind 0,25 bis 5,0 mol-% und besonders bevorzugt 0,5 bis 2,0 mol-%.

In einer weiteren bevorzugten Ausführungsform der Erfindung enthalten die erfindungsgemäßen Polymere

a) 45,0 bis 96,9 mol-%, vorzugsweise 57,1 bis 88,8 mol-% und besonders bevorzugt 72,5 bis 82,2 mol-% an einer oder mehreren wiederkehrenden Struktureinheiten der Formel (1),
b) 3,0 bis 50,0 mol-%, vorzugsweise 11,0 bis 39,9 mol-% und besonders bevorzugt 17,5 bis 25,0 mol-% an einer oder den mehreren wiederkehrenden Struktureinheiten der Formel (2), und
d) 0,1 bis 5,0 mol-%, vorzugsweise 0,2 bis 3,0 mol-% und besonders bevorzugt 0,3 bis 2,5 mol-% an einer oder mehreren wiederkehrenden vernetzenden Struktureinheiten der Formel (5).

In einer besonders bevorzugten Ausführungsform der Erfindung bestehen die wiederkehrenden Struktureinheiten der erfindungsgemäßen Polymere aus einer oder mehreren wiederkehrenden Struktureinheiten der Formel (1), einer oder mehreren wiederkehrenden Struktureinheiten der Formel (2) und einer oder mehreren wiederkehrenden Struktureinheiten der Formel (5). In einer insbesondere bevorzugten Ausführungsform der Erfindung sind die Mengen an Struktureinheiten der Formeln (1), (2) und (5) in den erfindungsgemäßen Polymeren dann wie soeben angegeben.

In einer weiteren bevorzugten Ausführungsform der Erfindung enthalten die erfindungsgemäßen Polymere 0,01 bis 30,0 mol-%, vorzugsweise 0,1 bis 20,0 mol-% und besonders bevorzugt 0,5 bis 15,0 mol-% an einer oder mehreren voneinander unabhängigen wiederkehrenden Struktureinheiten der Formel (3)

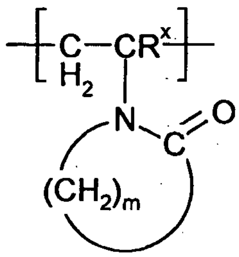

(3)

worin

$R^x$ Wasserstoff, Methyl oder Ethyl bedeutet und
m eine ganze Zahl von 2 bis 9 bedeutet.

In der einen oder den mehreren Struktureinheiten der Formel (3) ist $R^x$ vorzugsweise Wasserstoff oder Methyl und besonders bevorzugt Wasserstoff.

In der einen oder den mehreren Struktureinheiten der Formel (3) ist m vorzugsweise eine ganze Zahl von 2 bis 9, besonders bevorzugt eine ganze Zahl von 2 bis 6 und insbesondere bevorzugt ist m 3 oder 4.

In einer besonders bevorzugten Ausführungsform der Erfindung enthalten die erfindungsgemäßen Polymere Struktureinheiten der Formel (3) abgeleitet von N-Vinylpyrrolidon. Sofern die erfindungsgemäßen Polymere derartige Struktureinheiten enthalten, sind sie in einer darunter bevorzugten Ausführungsform der Erfindung in einer Menge von 0,5 bis 5,0 mol-% und in einer darunter besonders bevorzugten Ausführungsform der Erfindung in einer Menge von 0,75 bis 2,0 mol% in den erfindungsgemäßen Polymeren enthalten.

In einer weiteren bevorzugten Ausführungsform der Erfindung enthalten die erfindungsgemäßen Polymere 0,01 bis 30,0 mol-%, vorzugsweise 0,1 bis 20,0 mol-% und besonders bevorzugt 0,5 bis 15,0 mol% an einer oder mehreren voneinander unabhängigen wiederkehrenden Struktureinheiten der Formel (4)

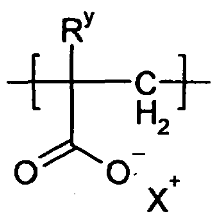

(4)

worin

$R^y$ Wasserstoff, Methyl oder Ethyl bedeutet und
$X^+$ für ein Gegenion steht.

In der einen oder den mehreren Struktureinheiten der Formel (4) ist $R^y$ vorzugsweise Wasserstoff oder Methyl und besonders bevorzugt Methyl.

In einer besonders bevorzugten Ausführungsform der Erfindung enthalten die erfindungsgemäßen Polymere eine oder mehrere Struktureinheiten der Formel (4) abgeleitet von Methacrylsäure oder ihren Salzen ($R^y$ = Methyl). Sofern die erfindungsgemäßen Polymere derartige Struktureinheiten enthalten, sind diese in einer darunter bevorzugten Ausführungsform der Erfindung in einer Menge von 0,5 bis 15,0 mol-% und in einer darunter besonders bevorzugten Ausführungsform der Erfindung in einer Menge von 1,75 bis 13,0 mol-% in den erfindungsgemäßen Polymeren enthalten.

In einem erfindunsgemäßen Polymer können jeweils verschiedene Struktureinheiten der Formel (3) und/oder der Formel (4) enthalten sein.

In einer weiteren bevorzugten Ausführungsform der Erfindung enthalten die erfindungsgemäßen Polymere eine oder mehrere Struktureinheiten der Formel (3) und eine oder mehrere Struktureinheiten der Formel (4).

In einer weiteren besonders bevorzugten Ausführungsform der Erfindung enthalten die erfindungsgemäßen Polymere Mischungen aus Struktureinheiten der Formel (3) abgeleitet von N-Vinylpyrrolidon und Struktureinheiten der Formel (4) abgeleitet von Methacrylsäure oder ihren Salzen. Sofern die erfindungsgemäßen Polymere Mischungen aus derartigen Struktureinheiten enthalten, sind diese Mischungen in einer darunter bevorzugten Ausführungsform der Erfindung in einer Menge von 1,5 bis 7,5 mol% und in einer darunter besonders bevorzugten Ausführungsform der Erfindung in einer Menge von 2,5 bis 6,5 mol-% in den erfind ungsgemäßen Polymeren enthalten.

Wie bereits erwähnt können in einem erfindungsgemäßen Polymer beispielsweise verschiedene Struktureinheiten der Formel (4) enthalten sein. Ein erfindungsgemäßes Polymer kann beispielsweise mehrere Struktureinheiten der Formel (4) enthalten, die sich durch unterschiedliche Gegenionen $X^+$ voneinander unterscheiden.

Vorzugsweise steht $X^+$ für $H^+$, $NH_4^+$, organische Ammoniumionen $[HNR^{5a}R^{6a}R^{7a}]^+$, wobei $R^{5a}$, $R^{6a}$ und $R^{7a}$ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste $R^{5a}$, $R^{6a}$ und $R^{7a}$ nicht Wasserstoff ist, $Alkali^+$, wobei unter $Alkali^+$ wiederum $Li^+$, $Na^+$ und $K^+$ bevorzugt sind, ½ $Erdalkali^{++}$, wobei unter ½ $Erdalkali^{++}$ wiederum ½ $Ca^{++}$ und ½ $Mg^{++}$ bevorzugt sind, ½ $Zn^{++}$ oder ⅓ $Al^{+++}$ oder für Mischungen aus diesen Ionen.

In der einen oder den mehreren Struktureinheiten der Formel (4) der erfindungsgemäßen Polymere ist das Gegenion $X^+$ besonders bevorzugt ausgewählt aus der Gruppe bestehend aus $H^+$, $NH_4^+$, $Alkali^+$, wobei unter $Alkali^+$ wiederum $Na^+$ bevorzugt ist, ½ $Erdalkali^{++}$, wobei unter ½ $Erdalkali^{++}$ wiederum ½ $Ca^{++}$ und ½ $Mg^{++}$ bevorzugt sind, und Mischungen aus diesen Ionen. Besonders bevorzugt ist das Gegenion $X^+$ ausgewählt aus der Gruppe bestehend aus $H^+$, $NH_4^+$ und $Na^+$ und insbesondere bevorzugt ist $X^+$ ausgewählt aus der Gruppe bestehend aus $H^+$ und $NH_4^+$.

In einer weiteren besonders bevorzugten Ausführungsform der Erfindung enthalten die erfindungsgemäßen Polymere mehrere Struktureinheiten der Formel (4), wobei in einigen Struktureinheiten der Formel (4) die Bedeutung der Gegenionen $X^+$ $H^+$ ist und in den anderen Struktureinheiten der Formel (4) die Bedeutung der Gegenionen $X^+$ eine andere als $H^+$ ist und $X^+$ in diesen anderen Struktureinheiten der Formel (4) vorzugsweise ausgewählt ist aus der Gruppe bestehend aus $NH_4^+$ und $Na^+$ und besonders bevorzugt $NH_4^+$ ist.

In einer insbesondere bevorzugten Ausführungsform der Erfindung ist das Gegenion $Q^+$ in der einen oder den mehreren Struktureinheiten der Formel (1) der erfindungsgemäßen Polymere ausgewählt aus der Gruppe bestehend aus $H^+$, $NH_4^+$, $Alkali^+$, wobei unter $Alkali^+$ wiederum $Na^+$ bevorzugt ist, ½ $Erdalkali^{++}$, wobei unter ½ $Erdalkali^{++}$ wiederum ½ $Ca^{++}$ und ½ $Mg^{++}$ bevorzugt sind, und Mischungen aus diesen Ionen, darunter bevorzugt ist $Q^+$ ausgewählt aus der Gruppe bestehend aus $H^+$, $NH_4^+$ und $Na^+$ und wiederum darunter bevorzugt ist $Q^+$ ausgewählt aus der Gruppe bestehend aus $H^+$ und $NH_4^+$ und in dieser insbesondere bevorzugten Ausführungsform der Erfindung ist das Gegenion $X^+$ in der einen oder den mehreren Struktureinheiten der Formel (4) der erfindungsgemäßen Polymere ausgewählt aus der Gruppe bestehend aus $H^+$, $NH_4^+$, $Alkali^+$, wobei unter $Alkali^+$ wiederum $Na^+$ bevorzugt ist, ½ $Erdalkali^{++}$, wobei unter ½ $Erdalkali^{++}$ wiederum ½ $Ca^{++}$ und ½ $Mg^{++}$ bevorzugt sind, und Mischungen aus diesen Ionen, darunter bevorzugt ist $X^+$ ausgewählt aus der Gruppe bestehend aus $H^+$, $NH_4^+$ und $Na^+$ und wiederum darunter bevorzugt ist $X^+$ ausgewählt aus der Gruppe bestehend aus $H^+$ und $NH_4^+$.

In einer weiteren besonders bevorzugten Ausführungsform der Erfindung ist $X^+$ in der einen oder den mehreren Struktureinheiten der Formel (4) $H^+$. Dies kann auch für den Fall gelten, dass $Q^+$ in der einen oder den mehreren Struktureinheiten der Formel (1) ein anderes Gegenion als $H^+$ ist oder zumindest teilweise ein anderes Gegenion als $H^+$ ist.

In einer weiteren bevorzugten Ausführungsform der Erfindung enthalten die erfindungsgemäßen Polymere

a) 25,0 bis 96,9 mol-%, vorzugsweise 42,0 bis 88,8 mol-% und besonders bevorzugt 64,5 bis 82,2 mol-% an einer oder mehreren wiederkehrenden Struktureinheiten der Formel (1),
b) 2,5 bis 50,0 mol-%, vorzugsweise 10,0 bis 39,9 mol-% und besonders bevorzugt 15,0 bis 25,0 mol-% an einer oder mehreren wiederkehrenden Struktureinheiten der Formel (2),
c) 0,5 bis 20,0 mol-%, vorzugsweise 1,0 bis 15,1 mol-% und besonders bevorzugt 2,0 bis 8,0 mol-% an einer oder mehreren wiederkehrenden Struktureinheiten der Formel (3) oder Formel (4) oder Mischungen aus einer oder mehreren wiederkehrenden Struktureinheiten der Formel (3) und Formel (4) und
d) 0,1 bis 5,0 mol-%, vorzugsweise 0,2 bis 3,0 mol-% und besonders bevorzugt 0,3 bis 2,5 mol% an einer oder mehreren wiederkehrenden vernetzenden Struktureinheiten der Formel (5).

In einer besonders bevorzugten Ausführungsform der Erfindung bestehen die wiederkehrenden Struktureinheiten der erfindungsgemäßen Polymere aus einer oder mehreren wiederkehrenden Struktureinheiten der Formel (1), einer oder mehreren wiederkehrenden Struktureinheiten der Formel (2), einer oder mehreren wiederkehrenden Struktureinheiten ausgewählt aus der Gruppe bestehend aus den Struktureinheiten der Formel (3) und der Formel (4) und einer oder mehreren wiederkehrenden Struktureinheiten der Formel (5). In einer insbesondere bevorzugten Ausführungsform der Erfindung sind die Mengen an Struktureinheiten der Formeln (1), (2), (5) und die Mengen an Struktureinheiten ausgewählt aus den Formeln (3) und (4) in den erfindungsgemäßen Polymeren dann wie soeben angegeben.

Sofern in den erfindungsgemäßen Polymeren Struktureinheiten der Formel (3) und der Formel (4) enthalten sind, ist das molare Verhältnis der einen oder der mehreren Struktureinheiten der Formel (3) zu der einen oder den mehreren Struktureinheiten der Formel (4) in einer darunter bevorzugten Ausführungsform der Erfindung von 1 : 99 bis 99 : 1, in einer darunter besonders bevorzugten Ausführungsform der Erfindung von 1 : 1 bis 1 : 20 und in einer darunter

insbesondere bevorzugten Ausführungsform der Erfindung von 1 : 2 bis 1 : 10.

In Polymeren enthaltene wiederkehrende Struktureinheiten gehen aus den zu ihrer Herstellung verwendeten polymerisierbaren Monomeren hervor. Die Verteilung der verschiedenen wiederkehrenden Struktureinheiten in den erfindungsgemäßen Polymeren kann statistisch, blockartig, alternierend oder gradientenartig sein und ist vorzugsweise statistisch oder gradientenartig.

Der Massenanteil an wiederkehrenden Struktureinheiten in den erfindungsgemäßen Polymeren, bezogen auf die Gesamtmasse aller in den erfindungsgemäßen Polymeren chemisch gebundenen Struktureinheiten, ist vorzugsweise größer oder gleich 85 Gew.-%, besonders bevorzugt größer oder gleich 90 Gew.-%, insbesondere bevorzugt größer oder gleich 95 Gew.-% und außerordentlich bevorzugt größer oder gleich 97 Gew.-%. Bei chemisch gebundenen Struktureinheiten, die in den erfindungsgemäßen Polymeren enthalten sein können, aber nicht aus den zu ihrer Herstellung verwendeten polymerisierbaren Monomeren hervorgehen, kann es sich insbesondere um Struktureinheiten handeln, die aus zur Herstellung der erfindungsgemäßen Polymere verwendeten Initiatoren hervorgehen.

In einer weiteren bevorzugten Ausführungsform der Erfindung sind die erfindungsgemäßen Polymere frei von Struktureinheiten der Formel (10)

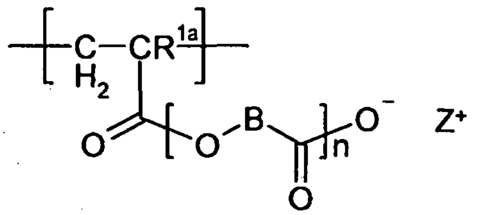

(10)

worin

- $R^{1a}$ Wasserstoff, Methyl oder Ethyl bedeutet,
- $Z^+$ für ein Gegenion und vorzugsweise für $H^+$, $NH_4^+$, organische Ammoniumionen $[HNR^{5b}R^{6b}R^{7b}]^+$, wobei $R^{5b}$, $R^{6b}$ und $R^{7b}$ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste $R^{5b}$, $R^{6b}$ und $R^{7b}$ nicht Wasserstoff ist, $Li^+$, $Na^+$, $K^+$, ½ $Ca^{++}$, ½ $Mg^{++}$, ½ $Zn^{++}$ oder ⅓ $Al^{+++}$ oder für Mischungen aus diesen Ionen steht,
- B eine lineare oder verzweigte Alkylengruppe mit 1 bis 6 Kohlenstoffatomen ist, und
- n eine ganze Zahl von 1 bis 10 ist.

In einer weiteren bevorzugten Ausführungsform der Erfindung enthalten die erfindungsgemäßen Polymere keine kationischen Struktureinheiten.

Die erfindungsgemäßen Polymere besitzen bevorzugt ein Molekulargewicht von $10^3$ bis $10^9$ g/mol, besonders bevorzugt von $10^4$ bis $10^7$ g/mol und insbesondere bevorzugt von $10^5$ bis $5 \cdot 10^6$ g/mol.

Die Herstellung der erfindungsgemäßen Polymere erfolgt mittels radikalischer Polymerisation in einem protischen Lösungsmittel, vorzugsweise in tert.-Butanol. Hierbei werden die entsprechenden Monomere z. B. in dem protischen Lösungsmittel gelöst oder dispergiert und die Polymerisation in an sich bekannter Weise, z. B. durch Zugabe einer radikalbildenden Verbindung, gestartet. Als Starter bzw. Initiator können im Prinzip alle für diesen Zweck bekannten und geeigneten Substanzen in den üblichen Mengen verwendet werden. In einer bevorzugten Ausführungsform der Erfindung werden jedoch Dilauroylperoxid oder Dimethyl 2,2'-azobis(2-methylpropionat) (V601) als Initiator verwendet. Die Menge an Initiator zur Herstellung der erfindungsgemäßen Polymere ist vorzugsweise kleiner oder gleich 10 Gew.-%, besonders bevorzugt kleiner oder gleich 5 Gew.-% und insbesondere bevorzugt kleiner oder gleich 3 Gew.-%, bezogen auf die Gesamtmenge aus zur Polymerisation verwendeten Monomeren und Initiator. Dabei können beispielsweise die vorgelegten Monomere "direkt" polymerisiert werden. Sie können aber auch vor der Polymerisation neutralisiert werden, indem beispielsweise saure Gruppen eingesetzter Monomere vor der Polymerisation mit Basen umgesetzt werden. Anstelle der Neutralisation der Monomere vor den Polymerisation können jedoch auch die Polymere nach der erfolgten Polymerisation mit den Basen neutralisiert werden.

Weiterer Gegenstand der vorliegenden Erfindung ist daher ein Verfahren zur Herstellung der erfindungsgemäßen Polymere, wobei Monomere, aus denen sich die Struktureinheiten der Formel (1), Formel (2) und Formel (5) ableiten, sowie gegebenenfalls weitere Monomere, aus denen sich beispielsweise die eine oder die mehreren Struktureinheiten ausgewählt aus den Formeln (3) und (4) ableiten, in einem protischen Lösungsmittel, vorzugsweise in tert.-Butanol, radikalisch polymerisiert werden, und gegebenenfalls die Monomere vor der Polymerisation oder das Polymer nach der

Polymerisation mit einer Base wie Ammoniak oder organischen Aminen oder einer Alkali$^+$-enthaltenden, vorzugsweise einer $Li^+$-, $Na^+$-oder $K^+$-enthaltenden, einer Erdalkali$^{++}$-enthaltenden, vorzugsweise einer $Ca^{++}$-oder $Mg^{++}$-enthaltenden, oder einer $Zn^{++}$- oder $Al^{+++}$-enthaltenden Base neutralisiert werden. Sofern eine Neutralisation mit Alkali$^+$-, Erdalkali$^{++}$-, $Zn^{++}$- oder $Al^{+++}$-enthaltenden Basen durchgeführt wird, wird sie in einer bevorzugten Ausführungsform mit den entsprechenden Hydroxiden oder Carbonaten und besonders bevorzugt mit Hydroxiden durchgeführt.

Radikalische Polymerisationen sind dem Fachmann allgemein bekannt und in Standardwerken der Literatur, z. B. in "Makromolekulare Chemie: Eine Einführung" von Bernd Tieke, Wiley-VCH, 2. vollständig überarbeitete und erweiterte Auflage (9. September 2005) ISBN-10: 3527313796 ausführlich beschrieben.

Die erfindungsgemäßen Polymere zeichnen sich durch eine gute Hautmilde und ein angenehmes, reichhaltiges Hautgefühl aus. Sie besitzen auch vorteilhafte Verdickungseigenschaften insbesondere in salzhaltigen Zusammensetzungen, wie z. B. in Wasser enthaltenden salzhaltigen Zusammensetzungen, und eine hohe Elektrolytstabilität. Die erfindungsgemäßen Polymere sind darüber hinaus säurestabil. Da die erfindungsgemäßen Polymere auch bei sauren ph-werten verdicken, kann man verdickte kosmetische, dermatologische oder pharmazeutische Produkte vorteilhafterweise auch mit organischen Säuren, wie Benzoesäure, Sorbinsäure, Paramethoxybenzoesäure konservieren, da auch bei den notwendigen niedrigen pH-Werten genügend Verdickerleistung zur Verfügung steht. Mit ihnen können klare Lösungen erhalten werden.

Die erfindungsgemäßen Polymere eignen sich in vorteilhafter Weise zur Herstellung von kosmetischen, dermatologischen oder pharmazeutischen Zusammensetzungen.

Weiterer Gegenstand der vorliegenden Erfindung ist daher die Verwendung eines oder mehrerer erfindungsgemäßer Polymere zur Herstellung von kosmetischen, dermatologischen oder pharmazeutischen Zusammensetzungen sowie kosmetische, dermatologische oder pharmazeutische Zusammensetzungen enthaltend ein oder mehrere erfindungsgemäße Polymere.

Die erfindungsgemäßen kosmetischen, dermatologischen oder pharmazeutischen Zusammensetzungen enthalten das eine oder die mehreren erfindungsgemäßen Polymere vorzugsweise in einer Menge von 0,01 bis 10,0 Gew.-%, besonders bevorzugt in einer Menge von 0,1 bis 5,0 Gew.-% und insbesondere bevorzugt in einer Menge von 0,25 bis 2,0 Gew.-%, jeweils bezogen auf das Gesamtgewicht der erfindungsgemäßen Zusammensetzungen.

In einer bevorzugten Ausführungsform der Erfindung haben die erfindungsgemäßen kosmetischen, dermatologischen oder pharmazeutischen Zusammensetzungen Viskositäten vorzugsweise im Bereich von 100 bis 200 000 mPa · s, besonders bevorzugt im Bereich von 1 000 bis 100 000 mPa · s, insbesondere bevorzugt im Bereich von 2 000 bis 50 000 mPa · s und außerordentlich bevorzugt im Bereich von 5 000 bis 30 000 mPa · s (25 °C, Brookfield RVT, T-C Spindel bei 20 Umdrehungen pro Minute).

In einer weiteren bevorzugten Ausführungsform der Erfindung liegen die erfindungsgemäßen Zusammensetzungen in Form von Fluids, Gelen, Schäumen, Sprays, Lotions oder Cremes vor.

Die erfindungsgemäßen Zusammensetzungen sind vorzugsweise auf wässriger oder wässrig-alkoholischer Basis aufgebaut oder liegen als Emulsionen, vorzugsweise als Öl-in-Wasser Emulsionen, vor.

In einer besonders bevorzugten Ausführungsform der Erfindung liegen die erfindungsgemäßen Zusammensetzungen als wässrig-alkoholische Zusammensetzungen vor und enthalten vorzugsweise, bezogen auf das Gesamtgewicht der Zusammensetzungen,

a) bis zu 90,0 Gew.-%, vorzugsweise 19,49 bis 80,0 Gew.-%, besonders bevorzugt 23,9 bis 70,0 Gew.-%, insbesondere bevorzugt 28,5 bis 60,0 Gew.-% Wasser,
b) bis zu 90,0 Gew.-%, vorzugsweise 19,49 bis 80,0 Gew.-%, besonders bevorzugt 28,9 bis 75,0 Gew.-%, insbesondere bevorzugt 38,5 bis 70,0 Gew.-% eines oder mehrerer Alkohole, vorzugsweise Ethanol oder Isopropanol,
c) bis zu 10,0 Gew.-%, vorzugsweise 0,01 bis 10,0 Gew.-%, besonders bevorzugt 0,1 bis 5,0 Gew.-%, insbesondere bevorzugt 0,5 bis 2,0 Gew.-% an einem oder mehreren der erfindungsgemäßen Polymere und
d) bis zu 20,0 Gew.-%, vorzugsweise 0,5 bis 10,0 Gew.-%, besonders bevorzugt 1,0 bis 5,0 Gew.-%, insbesondere bevorzugt 1,0 bis 3,0 Gew.-% eines oder mehrerer weiterer Zusatzstoffe.

Vorzugsweise ist der eine oder sind die mehreren weiteren Zusatzstoffe in den soeben genannten wässrig-alkoholischen Zusammensetzungen ausgewählt aus der Gruppe bestehend aus Tensiden und antimikrobiellen Wirkstoffen. In einer bevorzugten Ausführungsform der Erfindung, wie z. B. in dem soeben genannten Fall, liegen die erfindungsgemäßen Zusammensetzungen als Desinfektionsgele vor.

In einer weiteren besonders bevorzugten Ausführungsform der Erfindung liegen die erfindungsgemäßen Zusammensetzungen als Öl-in-Wasser Emulsionen vor und enthalten vorzugsweise, bezogen auf das Gesamtgewicht der Zusammensetzungen,

a) bis zu 95,0 Gew.-%, vorzugsweise 49,49 bis 95,0 Gew.-%, besonders bevorzugt 68,9 bis 90,0 Gew.-%, insbesondere bevorzugt 70,0 bis 85,0 Gew.-% einer Wasserphase oder wässrigen-alkoholischen Phase,

b) bis zu 70,0 Gew.-%, vorzugsweise 4,49 bis 50,0 Gew.-%, besonders bevorzugt 8,9 bis 30,0 Gew.-%, insbesondere bevorzugt 13,5 bis 25,0 Gew.-% einer Ölphase

c) bis zu 10,0 Gew.-%, vorzugsweise 0,01 bis 10,0 Gew.-%, besonders bevorzugt 0,1 bis 5,0 Gew.-%, insbesondere bevorzugt 0,5 bis 2,0 Gew.-% an einem oder mehreren der erfindungsgemäßen Polymere und

d) bis zu 20,0 Gew.-%, vorzugsweise 0,5 bis 10 Gew.-%, besonders bevorzugt 1,0 bis 5,0 Gew.-%, insbesondere bevorzugt 1,0 bis 3,0 Gew.-% eines oder mehrerer weiterer Zusatzstoffe.

Vorzugsweise ist der eine oder sind die mehreren weiteren Zusatzstoffe in den soeben genannten Öl-in-Wasser Emulsionen ausgewählt aus der Gruppe bestehend aus Emulgatoren, Co-Emulgatoren, Solubilisatoren, Aktivstoffen, Sonnenschutzfiltern, Pigmenten und antimikrobiellen Wirkstoffen.

Für die erfindungsgemäßen Zusammensetzungen auf wässrig-alkoholischer oder auch alkoholischer Basis kommen alle ein- oder mehrwertigen Alkohole in Betracht. Vorzugsweise werden Alkohole mit 1 bis 4 Kohlenstoffatomen wie Ethanol, Propanol, Isopropanol, n-Butanol, i-Butanol, tert.-Butanol oder Glycerin sowie Alkylenglykole, insbesondere Propylen-, Butylen- oder Hexylenglykol, und Mischungen aus den genannten Alkoholen verwendet. Weitere bevorzugte Alkohole sind Polyethylenglykole mit einer relativen Molekülmasse unter 2 000. Besonders bevorzugt ist ein Einsatz von Ethanol oder Isopropanol.

Die erfindungsgemäßen Zusammensetzungen können ein oder mehrere Öle enthalten.

Die Öle können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Methanol, Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, $C_8$-$C_{30}$-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol® 318. Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten $C_{8-22}$-Alkanolen, z. B. die Handelsprodukte Finsolv®SB (Isostearylbenzoat), Finsolv®TN ($C_{12}$-$C_{15}$-Alkylbenzoat) und Finsolv®EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol® OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert.-Butyl-n-octylether, iso-Pentyl-n-octylether und 2-Methyl-pentyl-n-octylether sowie Di-tert.-butylether und Di-isopentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zückersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von $C_{12}$-$C_{15}$-Fettalkoholen besonders bevorzugt. Ester dieses Typs sind im Handel erhältlich, z. B. unter dem Handelsnamen Cosmacol® der EniChem, Augusta Industriale.

Eine weitere Klasse von bevorzugten Ölkörpern sind Dicarbonsäureester von linearen oder verzweigten $C_2$-$C_{10}$-Alkanolen, wie Di-n-butyladipat (Cetiol® B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol® CC).

Eine weitere Klasse von bevorzugten polkörpern sind die Ester von Dimeren ungesättigter $C_{12}$-$C_{22}$-Fettsäuren (Dimerfettsäuren) mit einwertigen linearen, verzweigten oder cyclischen $C_2$-$C_{18}$-Alkanolen oder mit mehrwertig linearen oder verzweigten $C_2$-$C_6$-Alkanolen.

Eine weitere Klasse von bevorzugten Ölkörpern sind Kohlenwasserstofföle, zum Beispiel solche mit linearen oder verzweigten, gesättigten oder ungesättigten $C_7$-$C_{40}$-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der

Permethyl®-Serie, Squalan, Squalen, und alicyclische Kohlenwasserstoffe, z. B. das Handelsprodukt 1,3-Di-(2-ethylhexyl)-cyclohexan (Cetiol® S), Ozokerit, und Ceresin.

Ebenso in Betracht kommen Silikonöle bzw. -wachse, vorzugsweise Dimethylpolysiloxane und Cyclomethicone, Polydialkylsiloxane $R_3SiO(R_2SiO)_xSiR_3$, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare® Silicone 41 M65, SilCare® Silicone 41M70, SilCare® Silicone 41M80 (Clariant) erhältlichen Dimethicone, Stearyldimethylpolysiloxan, $C_{20}$-$C_{24}$-Alkyl-dimethylpolysiloxan, $C_{24}$-$C_{28}$-Alkyl-dimethylpolysiloxan, aber auch die unter SilCare® Silicone 41M40, SilCare® Silicone 41M50 (Clariant) erhältlichen Methicone, weiterhin Trimethylsiloxysilicate $[(CH_2)_3SiO)_{1/2}]_x[SiO_2]_y$, wobei x für eine Zahl von 1 bis 500 und y für eine Zahl von 1 bis 500 steht, Dimethiconole $R_3SiO[R_2SiO]_xSiR_2OH$ und $HOR_2SiO[R_2SiO]_xSiR_2OH$, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen Zusammensetzungen können als weitere Hilfs- und Zusatzstoffe beispielsweise Wachse, Emulgatoren, Co-Emulgatoren, Solubilisatoren, Elektrolyte, Hydroxysäuren, Stabilisatoren, kationische Polymere, Filmbildner, weitere Verdicker, Gelierungsmittel, Überfettungsmittel, Rückfetter, antimikrobielle Wirkstoffe, biogene Wirkstoffe, Adstringentien, deodorierende Stoffe, Sonnenschutzfilter, Antioxidantien, Feuchthaltemittel, Lösungsmittel, Farbmittel, Perlglanzmittel, Duftstoffe, Trübungsmittel und/oder Silikone enthalten.

Die erfindungsgemäßen Zusammensetzungen können Wachse, beispielsweise Paraffinwachse, Mikrowachse und Ozokerite, Bienenwachs und ihre Teilfraktionen sowie Bienenwachsderivate, Wachse aus der Gruppe der homopolymeren Polyethylene oder Copolymere der $\alpha$-Olefine, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden,

Als nichtionogene oberflächenaktive Verbindungen kommen vorzugsweise in Betracht:

Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; ($C_{12}$-$C_{18}$)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetyfether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearyiether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether.

Ebenso bevorzugt sind Fettsäureethoxylate, gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethyfenglykol(21)isostearat, Polyethylenglykol(22)isostearat, Pölyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)ofeat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders Polyethylenglykol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

Besonders vorteilhafte Coemulgatoren sind Glycerylmonostearät, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol (ABIL® EM 90), Laurylmethiconcopolyol oder Amodimethicone Glycerocarbamate (SilCare® Silicone WSI, Clariant).

Sofern die erfindungsgemäßen Zusammensetzungen eine oder mehrere Substanzen ausgewählt aus der Gruppe bestehend aus Emulgatoren, Co-Emulgatoren und Solubilisatoren enthalten, ist diese eine oder sind diese mehreren Substanzen, bezogen auf das Gesamtgewicht der entsprechenden erfindungsgemäßen Zusammensetzung, vorzugsweise in einer Menge von 0,1 bis 20,0 Gew.-%, besonders bevorzugt in einer Menge von 0,5 bis 10,0 Gew.-% und insbesondere bevorzugt in einer Menge von 1,0 bis 5,0 Gew.-% in den erfindungsgemäßen Zusammensetzungen enthalten.

Als Elektrolyt zum Einsatz können kommen anorganische Salze, bevorzugt Ammonium- oder Metallsalze, besonders bevorzugt von Halogeniden, beispielsweise $CaCl_2$, $MgCl_2$, LiCl, KCl und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure.

Hierzu zählen auch Aluminiumsalze, bevorzugt Aluminiumchlorohydrat oder Aluminium-Zirkonium-Komplexsalze.

In einer bevorzugten Ausführungsform der Erfindung enthalten die erfindungsgemäßen Zusammensetzungen daher ein oder mehrere Substanzen ausgewählt aus anorganischen und organischen Salzen.

Als Elektrolyt können die erfindungsgemäßen Zusammensetzungen auch Mischungen verschiedener Salze enthalten.

Sofern die erfindungsgemäßen Zusammensetzungen einen oder mehrere Elektrolyte enthalten, sind diese, bezogen auf das Gesamtgewicht der entsprechenden erfindungsgemäßen Zusammensetzung, vorzugsweise in einer Menge von 0,01 bis 20,0 Gew.-%, besonders bevorzugt in einer Menge von 0,1 bis 10,0 Gew.-% und insbesondere bevorzugt in einer Menge von 0,5 bis 5,0 Gew.-% in den erfindungsgemäßen Zusammensetzungen enthalten.

Die erfindungsgemäßen Polymere sind säurestabil und eignen sich vorzugsweise zur Verwendung in kosmetischen, pharmazeutischen und/oder dermatologischen Zusammensetzungen mit niedrigem pH-Wert von 2 bis 6, insbesondere für Produkte zur Hand- und Hautdesinfektion sowie zur Hautpflege.

Die Verwendung von sauren Additiven und deren Salzen macht es teilweise notwendig, den pH-Wert der kosmetischen oder dermatologischen Zusammensetzungen in einen deutlich sauren Bereich einzustellen.

In einer weiteren bevorzugten Ausführungsform der Erfindung enthalten die erfindungsgemäßen Zusammensetzungen ein oder mehrere Hydroxysäuren, besonders bevorzugt ein oder mehrere Substanzen ausgewählt aus alpha-

und beta-Hydroxysäuren.

An Hydroxysäuren können die erfindungsgemäßen Zusammensetzungen vorzugsweise Milchsäure, Glykolsäure, Salicylsäure und alkylierte Salicylsäuren oder Zitronensäure enthalten. Weiterhin können erfindungsgemäße Formulierungen weitere saure Komponenten enthalten. Als Wirkstoff in Betracht kommen Weinsäure, Mandelsäure, Caffeic acid, Brenztraubensäure, Oligooxa Mono- und Dicarbonsäuren, Fumarsäure, Retinoesäure, Sulfonsäuren, Benzoesäure, Kojisäure, Fruchtsäure, Äpfelsäure, Gluconsäure, Pyruvic Acid, Galacturonic Acid, Ribonic Acid, und all deren Derivate, Polyglykoldisäuren in freier oder teilweiser neutralisierter Form, Vitamin C (Ascorbinsäure), Vitamin C-Derivate, Dihydroxyaceton oder Skin-whitening Actives wie Arbutin oder Glycyrrhetinsäure und deren Salze. Sofern die erfindungsgemäßen Zusammensetzungen eine oder mehrere dieser soeben genannten Substanzen enthalten, ist diese eine oder sind diese mehreren Substanzen, bezogen auf das Gesamtgewicht der entsprechenden erfindungsgemäßen Zusammensetzung, vorzugsweise in einer Menge von 0,1 bis 20,0 Gew.-%, besonders bevorzugt in einer Menge von 0,2 bis 10,0 Gew.-% und insbesondere bevorzugt in einer Menge von 0,5 bis 5,0 Gew.-% in den erfindungsgemäßen Zusammensetzungen enthalten.

In einer weiteren bevorzugten Ausführungsform der Erfindung enthalten die erfindungsgemäßen Zusammensetzungen daher ein oder mehrere Substanzen ausgewählt aus Vitamin C und Vitamin C-Derivaten, wobei die Vitamin C-Derivate vorzugsweise ausgewählt sind aus Natriumascorbylphosphat, Magnesiumascorbylphoshat und Magnesiumascorbylglucosid.

In einer weiteren bevorzugten Ausführungsform der Erfindung enthalten die erfindungsgemäßen Zusammensetzungen eine oder mehrere Substanzen ausgewählt aus Benzoesäure, Sorbinsäure, Salicylsäure, Milchsäure und Paramethoxybenzoesäure. Dadurch, dass die erfindungsgemäßen Polymere auch im sauren pH-Bereich verdicken und eine Fließgrenze aufbauen, kann man mit den oben genannten organischen Säuren als Konservierungsmittel arbeiten.

Zusätzlich zu den erfindungsgemäßen Polymeren können als zusätzliche Stabilisatoren Metallsalze von Fettsäuren, wie z. B. Magnesium-, Aluminium- und/oder Zinkstearat eingesetzt werden. Sofern die erfindungsgemäßen Zusammensetzungen eine oder mehrere dieser soeben genannten Substanzen enthalten, ist diese eine oder sind diese mehreren Substanzen, bezogen auf das Gesamtgewicht der entsprechenden erfindungsgemäßen Zusammensetzung, vorzugsweise in einer Menge von 0,1 bis 10,0 Gew.-%, besonders bevorzugt in einer Menge von 0,5 bis 8,0 Gew.-% und insbesondere bevorzugt in einer Menge von 1,0 bis 5,0 Gew.-% in den erfindungsgemäßen Zusammensetzungen enthalten.

Als kationische Polymere eignen sich die unter der INCI-Bezeichnung "Polyquaternium" bekannten Substanzen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxy-propyldiethylentriamin; Polyaminopolyamid und kationische Chitinderivate, wie beispielsweise Chitosan.

Sofern die erfindungsgemäßen Zusammensetzungen ein oder mehrere der oben genannten kationischen Polymere enthalten, sind diese, bezogen auf das Gesamtgewicht der entsprechenden erfindungsgemäßen Zusammensetzung, vorzugsweise in einer Menge von 0,1 bis 5,0 Gew.-%, besonders bevorzugt in einer Menge von 0,2 bis 3,0 Gew.-% und insbesondere bevorzugt in einer Menge von 0,5 bis 2,0 Gew.-% in den erfindungsgemäßen Zusammensetzungen enthalten.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise $C_{10}$-Polycarbamylpolyglycerylester, Polyvinylalkohol, Polyvinylpyrrolidoncopolymeren, beispielsweise Vinylpyrrolidon/Vinylacetatcopolymer oder PVP/Eicosene-Copolymeren, maleinierten Polypropylenpolymeren, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex® A 60 (Clariant) erhältlich.

Sofern die erfindungsgemäßen Zusammensetzungen einen oder mehrere Filmbildner enthalten, sind diese, bezogen auf das Gesamtgewicht der entsprechenden erfindungsgemäßen Zusammensetzung, vorzugsweise in einer Menge von 0,1 bis 10,0 Gew.-%, besonders bevorzugt in einer Menge von 0,2 bis 5,0 Gew.-% und insbesondere bevorzugt in einer Menge von 0,5 bis 3,0 Gew.-% in den erfindungsgemäßen Zusammensetzungen enthalten.

Die gewünschte Viskosität der Zusammensetzungen kann durch Zugabe von weiteren Verdickern und Gelierungsmittel eingestellt werden. In Betracht kommen vorzugsweise Celluloseether und andere Cellulosederivate (z. B.

Carboxymethylcellulöse, Hydroxyethylcellulose), Gelatine, Stärke und Stärkederivate, Natriumalginate, Fettsäurepolyethylenglykolester, Agar-Agar, Caragenan, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammonium Acryloyldimethyltaurate/VP-Copolymer oder Sodium Acryloyldimethyltaurate/VP-Copolymer Verwendung finden.

Sofern die erfindungsgemäßen Zusammensetzungen eine oder mehrere Substanzen ausgewählt aus der Gruppe bestehend aus Verdickern und Geliermitteln enthalten, ist diese eine oder sind diese mehreren Substanzen, bezogen auf das Gesamtgewicht der entsprechenden erfindungsgemäßen Zusammensetzung, vorzugsweise in einer Menge von 0,01 bis 20,0 Gew.-%, besonders bevorzugt in einer Menge von 0,1 bis 10,0 Gew.-%, insbesondere bevorzugt in einer Menge von 0,2 bis 3,0 Gew.-% und außerordentlich bevorzugt in einer Menge von 0,4 bis 2,0 Gew.-% in den erfindungsgemäßen Zusammemnsetzungen enthalten.

Als Überfettungsmittel oder Rückfetter können vorzugsweise Lanolin und Lecithin, nicht ethoxylierte und polyethoxylierte oder acylierte Lanolin- und Lecithinderivate, Polyolfettsäureester wie Glyceryloleat, Mono-, Di- und Triglyceride und/oder Fettsäurealkanolamide, wobei die letzteren gleichzeitig als Schaumstabilisatoren dienen, verwendet werden. Sofern die erfindungsgemäßen Zusammensetzungen eine oder mehrere der soeben genannten Substanzen enthalten, ist diese eine oder sind diese mehreren Substanzen, bezogen auf das Gesamtgewicht der entsprechenden erfindungsgemäßen Zusammensetzung, vorzugsweise in einer Menge von 0,01 bis 10,0 Gew.-%, besonders bevorzugt in einer Menge von 0,1 bis 5,0 Gew.-% und insbesondere bevorzugt in einer Menge von 0,5 bis 3,0 Gew.-% in den erfindungsgemäßen Zusammensetzungen enthalten.

In einer weiteren bevorzugten Ausführungsform der Erfindung enthalten die erfindungsgemäßen Zusammensetzungen ein oder mehrere antimikrobielle Wirkstoffe und liegen vorzugsweise in der Form von Desinfektionszusammensetzungen und besonders bevorzugt in der Form von Desinfektionsgelen vor.

An antimikrobiellen Wirkstoffen können Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox®, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz kommen. Sofern die erfindungsgemäßen Zusammensetzungen einen oder mehrere antimikrobielle Wirkstoffe enthalten, sind diese, bezogen auf das Gesamtgewicht der entsprechenden erfindungsgemäßen Zusammensetzung, vorzugsweise in einer Menge von 0,001 bis 5,0 Gew.-%, besonders bevorzugt in einer Menge von 0,01 bis 3,0 Gew.-% und insbesondere bevorzugt in einer Menge von 0,1 bis 2,0 Gew.-% in den erfindungsgemäßen Zusammensetzungen enthalten.

Die erfindungsgemäßen Zusammensetzungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol®, Allantoin, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Sofern die erfindungsgemäßen Zusammensetzungen einen oder mehrere biogene Wirkstoffe enthalten, dind diese, bezogen auf das Gesamtgewicht der entsprechenden erfindungsgemäßen Zusammensetzung, vorzugsweise in einer Menge von 0,001 bis 5,0 Gew.-%, besonders bevorzugt in einer Menge von 0,01 bis 3,0 Gew.-% und insbesondere bevorzugt in einer Menge von 0,1 bis 2,0 Gew.-% in den erfindungsgemäßen Zusammensetzungen enthalten.

Die erfindungsgemäßen Zusammensetzungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate enthalten. Sofern die erfindungsgemäßen Zusammensetzungen ein oder mehrere Adstringentien enthalten, sind diese, bezogen auf das Gesamtgewicht der entsprechenden erfindungsgemäßen Zusammensetzung, vorzugsweise in einer Menge von 0,001 bis 50,0 Gew.-%, besonders bevorzugt in einer Menge von 0,01 bis 10,0 Gew.-% und insbesondere bevorzugt in einer Menge von 0,1 bis 10,0 Gew.-% in den erfindungsgemäßen Zusammensetzungen enthalten.

Als deodorierende Stoffe bevorzugt sind Allantoin und Bisabolol. Sofern die erfindungsgemäßen Zusammensetzungen ein oder mehrere deodorierende Stoffe enthalten, sind diese, bezogen auf das Gesamtgewicht der entsprechenden erfindungsgemäßen Zusammensetzung, vorzugsweise in einer Menge von 0,0001 bis 10,0 Gew.-% in den erfindungsgemäßen Zusammensetzungen enthalten.

In einer weiteren bevorzugten Ausführungsform der Erfindung enthalten die erfindungsgemäßen Zusammensetzungen ein oder mehrere Substanzen ausgewählt aus anorganischen und organischen UV-Filtern und liegen besonders bevorzugt in der Form von Sonnenschutzzusammensetzungen vor.

Die erfindungsgemäßen Zusammensetzungen können als Pigmente/Mikropigmente sowie als anorganische Sonnenschutzfilter bzw. UV-Filter mikrofeines Titandioxid, Glimmer-Titanoxid, Eisenoxide, Glimmer-Eisenoxid, Zinkoxid, Siliciumoxide, Ultramarinblau oder Chromoxide enthalten.

Die organischen Sonnenschutzfilter bzw. UV-Filter sind vorzugsweise ausgewählt aus 4-Aminobenzoesäure, 3-(4'-Trimethylammonium)-benzyliden-boran-2-onmethylsulfat, Camphor Benzalkonium Methosulfat, 3,3,5-Trimethylcyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäureisoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-aminocarbonyl)phenylamino]-1,3,5-triazin-2,4-yl)diimino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-{2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzim idazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2-Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Sofern die erfindungsgemäßen Zusammensetzungen einen oder mehrere Sonnenschutzfilter enthalten, sind diese, bezogen auf das Gesamtgewicht der entsprechenden erfindungsgemäßen Zusammensetzung, vorzugsweise in einer Menge von 0,001 bis 30,0 Gew.-%, besonders bevorzugt in einer Menge von 0,05 bis 20,0 Gew.-% und insbesondere bevorzugt in einer Menge von 1,0 bis 10,0 Gew.-% in den erfindungsgemäßen Zusammensetzungen enthalten.

Die erfindungsgemäßen Zusammensetzungen können ein oder mehrere Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B.

Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten a-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, $ZnSO_4$), Selen und dessen Derivaten (z. B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Sofern die erfindungsgemäßen Zusammensetzungen einen oder mehrere Antioxidantien enthalten, sind diese, bezogen auf das Gesamtgewicht der entsprechenden erfindungsgemäßen Zusammensetzung, vorzugsweise in einer Menge von 0,001 bis 30,0 Gew.-%, besonders bevorzugt in einer Menge von 0,05 bis 20,0 Gew.-% und insbesondere bevorzugt in einer Menge von 1,0 bis 10,0 Gew.-% in den erfindungsgemäßen Zusammensetzungen enthalten.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxyethylharnstoff, Hydroxysäuren, Panthenol und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat, Glycerin und/oder Sorbitol eingesetzt werden. Sofern die erfindungsgemäßen Zusammensetzungen ein oder mehrere Feuchthaltemittel enthalten, sind diese, bezogen auf das Gesamtgewicht der entsprechenden erfindungsgemäßen Zusammensetzung, vorzugsweise in einer Menge von 0,1 bis 15,0 Gew.-% und besonders bevorzugt in einer Menge von 0,5 bis 5,0 Gew.-% in den erfindungsgemäßen Zusammensetzungen enthalten.

Zusätzlich können die erfindungsgemäßen Zusammensetzungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, tert.-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Die erfindungsgemäßen Zusammensetzungen können ein oder mehrere Substanzen ausgewählt aus Farbmitteln, z. B. Farbstoffe und/oder Pigmente, enthalten. Die in den erfindungsgemäßen Formulierungen enthaltenen Farbstoffe und/oder Pigmente, sowohl organische als auch anorganische Farbstoffe und Pigmente, sind aus der entsprechenden Positivliste der Kosmetikverordnung bzw. der EG-Liste kosmetischer Färbemittel ausgewählt.

| Chemische oder sonstige Bezeichnung | CIN | Farbe |
|---|---|---|
| Pigment Green | 10006 | Grün |
| Acid Green 1 | 10020 | Grün |
| 2,4-Dinitrohydroxynaphtalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |

(fortgesetzt)

| Chemische oder sonstige Bezeichnung | CIN | Farbe |
|---|---|---|
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hydroxy-5"-chloro-2",4"-dimethoxy-2-naphthoesäureanilid | 12490 | Rot |
| Disperse Yellow 16 | 12700 | Gelb |
| 1 -(4-Sulfo-1-phenylazo)-4-amino-brezol-sulfosäure | 13015 | Gelb |
| 2,4-Dihydroxy-azobenzol-4'-sulfosäure | 14270 | Orange |
| 2-(2,4-Dimethylphenylazo-5-sulfosäurse)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8,-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |

(fortgesetzt)

| Chemische oder sonstige Bezeichnung | CIN | Farbe |
|---|---|---|
| 4-(4-Sulfo-1-phenylazo)-1-(4-sulfophenyl)-5-hydroxy-phrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"Sulfo-1"-phenylazo)-7'-sulfo-1'-naphthylazo]-1-hydroxy-7-aminoaphthalin-3,6-disulfosäure | 27755 | Schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino-phenyl-(2-sulfophenyl)-methylen-(N-ethyl-N-p-sulfo-benzyl)-cyclohexadienimin | 42090 | Blau |
| Acid Green 9 | 42100 | Grün |
| Diethyl-di-sulfobenzyl-di-4-amino-2-chlor-di-2-methyl-fuchsonimonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | Blau |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylaminonaphtofuchsinimmonium | 44090 | Grün |
| Acid red | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |

(fortgesetzt)

| Chemische oder sonstige Bezeichnung | CIN | Farbe |
|---|---|---|
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid violet 50 | 50325 | Violett |
| Acid Black 2 | 50420 | Schwarz |
| Pigment Violet 23 | 51319 | Violett |
| 1,2-Dioxyanthrachinon, Calcium-Aluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidine)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | Orange |
| Indigo | 73000 | Blau |
| Indigo-disulfosäure | 73015 | Blau |
| 4,4'-Dimethyl-6,6'-dichlorthioindigo | 73360 | Rot |
| 5,5'-Dichlor-7,7'-dimethylthioindigo | 73385 | Violett |
| Quinacridone Violet 19 | 73900 | Violett |
| Pigment Red 122 | 73915 | Rot |
| Pigment Blue 16 | 74100 | Blau |
| Phthalocyanine | 74160 | Blau |
| Direct Blue 86 | 74180 | Blau |
| chlorierte Phthalocyanine | 74260 | Grün |
| Natural Yellow 6,19; Natural Red 1 | 75100 | Gelb |
| Bixin, Nor-Bixin | 75120 | Orange |
| Lycopin | 75125 | Gelb |
| trans-alpha, beta- bzw. gamma-Carotin | 75130 | Orange |
| Keto- und/oder Hydroxylderivate des Carotins | 75135 | Gelb |

(fortgesetzt)

| Chemische oder sonstige Bezeichnung | CIN | Farbe |
|---|---|---|
| Guanin oder Perlglanzmittel | 75170 | Weiß |
| 1,7-Bis-(4-hydroxy-3-methoxyphenyl)1,6-heptadien-3,5-dion | 75300 | Gelb |
| Komplexsalz (Na,Al,Ca) der Karminsäure | 75470 | Rot |
| Chlorophyll a und b; Kupferverbindungen der Chlorophylle und Chlorophylline | 75810 | Grün |
| Aluminium | 77000 | Weiß |
| Tonerdehydrat | 77002 | Weiß |
| wasserhaltige Aluminiumsilikate | 77004 | Weiß |
| Ultramarin | 77007 | Blau |
| Pigment Red 101 und 102 | 77015 | Rot |
| Bariumsulfat | 77120 | Weiß |
| Bismutoxychlorid und seine Gemische mit Glimmer | 77163 | Weiß |
| Calciumcarbonat | 77220 | Weiß |
| Calciumsulfat | 77231 | Weiß |
| Kohlenstoff | 77266 | Schwarz |
| Pigment Black 9 | 77267 | Schwarz |
| Carbo medicinalis vegetabilis | 77268:1 | Schwarz |
| Chromoxid | 77288 | Grün |
| Chromoxid, wasserhaltig | 77289 | Grün |
| Pigment Blue 28, Pigment Green 14 | 77346 | Grün |
| Pigment Metal 2 | 77400 | Braun |
| Gold | 77480 | Braun |
| Eisenoxide und -hydroxide | 77489 | Orange |
| Eisenoxide und -hydroxide | 77491 | Rot |
| Eisenoxidhydrat | 77492 | Gelb |
| Eisenoxid | 77499 | Schwarz |
| Mischungen aus Eisen(II)- und Eisen(III)-hexacyanoferrat | 77510 | Blau |
| Pigment White 18 | 77713 | Weiß |
| Mangananimoniumdiphosphat | 77742 | Violett |
| Manganphosphat; $Mn_3(PO_4)_2$*$7H_2O$ | 77745 | Rot |
| Silber | 77820 | Weiß |
| Titandioxid und seine Gemische mit Glimmer | 77891 | Weiß |
| Zinkoxid | 77947 | Weiß |
| 6,7-Dimethyl-9-(1'-D-ribityl)-isoalloxazin, Lactoflavin | | Gelb |
| Zuckerkulör | | Braun |
| Capsanthin, Capsorubin | | Orange |
| Betanin | | Rot |
| Benzopyriliumsalze, Anthocyanine | | Rot |
| Aluminium-, Zink-, Magnesium-, und Calciumstearat | | Weiß |

(fortgesetzt)

| Chemische oder sonstige Bezeichnung | CIN | Farbe |
|---|---|---|
| Bromthymolblau | | Blau |
| Bromkresolgrün | | Grün |
| Acid Red 195 | | Rot |

Ferner vorteilhaft sind öllösliche Naturfarbstoffe, wie z. B. Paprikaextrakte, β-Carotin und Cochenille.

Vorteilhaft eingesetzt werden auch Perlglanzpigmente, z. B. Fischsilber (Guanin/ Hypoxanthin-Mischkristalle aus Fischschuppen) und Perlrnutt (vermahlene Muschelschalen), monokristalline Perlglanzpigmente wie z. B. Bismuthoxychlorid (BiOCl), Schicht-Substrat Pigmente, z. B. Glimmer/Metalloxid, silberweiße Perlglanzpigmente aus $TiO_2$, Interferenpigmente ($TiO_2$, unterschiedliche Schichtdicke), Farbglanzpigmente ($Fe_2O_3$) und Kombinationspigmente ($TiO_2/Fe_2O_3$, $TiO_2/Cr_2O_3$, $TiO_2$/Berliner Blau, $TiO_2$/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere $SiO_2$-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCl), sowie die Oxide von beispielsweise Titan, insbesondere die $TiO_2$-Modifikatione Anatas und Rutil, Aluminium, Tantal, Niob, Zirkon und Hafnium. Auch mit Magnesiumfluorid ($MgF_2$) und Calciumfluorid (Flussspat, $CaF_2$) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2 - 50 μm, 5 - 25 μm, 5 - 40 μm, 5 - 60 μm, 5 - 95 μm, 5 - 100 μm, 10 - 60 μm, 10 - 100 μm, 10 - 125 μm, 20 - 100 μm, 20 - 150 μm, sowie < 15 μm. Eine breitere Teilchengrößenverteilung z. B. von 20 - 150 μm, ruft glitzernde Effekte hervor, während eine engere Teilcherigrößenverteilung von < 15 μm für eine gleichmäßige seidige Erscheinung sorgt.

Sofern die erfindungsgemäßen Zusammensetzungen ein oder mehrere Effektpigmente einthalten, sind diese, bezogen auf das Gesamtgewicht der entsprechenden erfindungsgemäßen Zusammensetzung, vorzugsweise in einer Menge von 0,1 bis 20,0 Gew.-%, besonders bevorzugt in einer Menge von 0,5 bis 10,0 Gew.-% und insbesondere bevorzugt in einer Menge von 1,0 bis 5,0 Gew.-% in den erfindungsgemäßen Zusammensetzungen enthalten.

Als perlglanzgebende Komponente bevorzugt geeignet sind

Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen.

Besonders bevorzugt sind Ethylenglykoldistearate und/oder Polyethylenglykoldistearate mit durchschnittlich 3 Glykoleinheiten.

Sofern die erfindungsgemäßen Zusammensetzungen eine oder mehrere perlglanzgebende Verbindungen enthalten, sind diese, bezogen auf das Gesamtgewicht der entsprechenden erfindungsgemäßen Zusammensetzung, vorzugsweise in einer Menge von 0,1 bis 15,0 Gew.-% und besonders bevorzugt in einer Menge von 1,0 bis 10,0 Gew.-% in den erfindungsgemäßen Zusammensetzungen enthalten.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat; Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Trübungsmittel können Polymerdispersionen insbesondere Polyacrylatderivat-, Polyacrylamidderivat-, Poly(acrylatderivat-co-acrylamidderivat)-Dispersionen, Poly(styrolderivate-co-acrylatderivat)-Dispersionen, gesättigte und ungesättigte Fettalkohole verwendet werden.

An Silikonen können die zuvor unter den Silikonölen bzw. -wachsen genannten Substanzen verwendet werden.

Als Säuren oder Laugen zur pH-Wert Einstellung können vorzugsweise Mineralsäuren, insbesondere HCl, anorganische Basen, insbesondere NaOH oder KOH, und organische Säuren, insbesondere Zitronensäure, verwendet werden.

Die erfindungsgemäßen Zusammensetzungen besitzen pH-Werte von vorzugsweise 2 bis 10, besonders bevorzugt von 2 bis 7 und insbesondere bevorzugt von 2,5 bis 6,5.

Die erfindungsgemäßen Polymere sind in vorteilhafter Weise als Verdicker, Konsistenzgeber, Emulgator, Sensorikadditiv, Solubilisator, Dispergator, Gleitmittel, Haftmittel, Stabilisator oder Fließgrenzenbildner geeignet.

Ein weiterer Gegenstand der Erfindung betrifft daher die Verwendung eines oder mehrerer der erfindungsgemäßen Polymere als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Sensorikadditiv, besonders bevorzugt als Verdicker oder Sensorikadditiv und insbesondere bevorzugt als Sensorikadditiv, außerordentlich bevorzugt in kosmetischen, dermatologischen oder pharmazeutischen Zusammensetzungen.

In einer weiteren bevorzugten Ausführungsform der Erfindung werden die erfindungsgemäßen Polymere zur Stabilisierung von Emulsionen, vorzugsweise von salzhaltigen Emulsionen und besonders bevorzugt von salzhaltigen kosmetischen, dermatologischen oder pharmazeutischen Emulsionen, verwendet.

Die erfindungsgemäßen Polymere sind säurestabil und eignen sich in vorteilhafter Weise zur Verwendung in kosmetischen, dermatologischen oder pharmazeutischen Zusammensetzungen mit niedrigem pH-Wert, insbesondere für die Pflege und Behandlung der Körper- oder Gesichtshaut.

Weiterer Gegenstand der vorliegenden Erfindung ist daher die Verwendung eines oder mehrerer erfindungsgemäßer Polymere zur Pflege und Behandlung der Körper- oder Gesichtshaut, vorzugsweise in kosmetischen, dermatologischen oder pharmazeutischen Zusammensetzungen, besonders bevorzugt in sauren kosmetischen, dermatologischen oder pharmazeutischen Zusammensetzungen.

Weiterhin vorteilhaft ist, dass die erfindungsgemäßen Polymere auch ohne Mitverwendung eines zusätzlichen Sensorikadditivs und/oder ohne Mitverwendung eines zusätzlichen Verdickers in Zusammensetzungen, vorzugsweise in kosmetischen, dermatologischen oder pharmazeutischen Zusammensetzungen, eingesetzt werden können. Die Mitverwendung von zusätzlichen Sensorikadditiven und/oder Verdickern ist daher nicht zwingend, aber möglich. Eine Kombination mit anderen bekannten Sensorikadditiven und/oder Verdickern kann zur Einstellung spezieller kosmetischer Profile und zur Ausnutzung synergistischer Effekte wünschenswert sein. Vorzugsweise liegen diese Zusammensetzungen als wässrige, wässrig-alkoholische, wässrig-tensidhaltige, wässrig-alkoholische tensidhaltige kosmetische, dermatologische oder pharmazeutische Zusammensetzungen vor, wobei diese vorzugsweise keine zusätzliche Substanz ausgewählt aus Sensorikadditiven und zusätzlichen Verdickern enthalten.

Wie bereits erwähnt enthalten die erfindungsgemäßen Zusammensetzungen in einer weiteren bevorzugten Ausführungsform der Erfindung ein oder mehrere Substanzen ausgewählt aus anorganischen und organischen UV-Filtern und liegen besonders bevorzugt in der Form von Sonnenschutzzusammensetzungen vor. Die erfindungsgemäßen Polymere weisen dabei den Vorteil auf, dass sie den Sonnenschutzfaktor der Sonnenschutzzusammensetzungen erhöhen können.

Weiterer Gegenstand der vorliegenden Erfindung ist daher auch die Verwendung eines oder mehrerer der erfindungsgemäßen Polymere zur Erhöhung des Sonnenschutzfaktors von Sonnenschutzzusammensetzungen.

Die nachfolgenden Beispiele und Anwendungen sollen die Erfindung näher erläutern, ohne sie jedoch darauf zu beschränken.

A) Beispiele zur Herstellung erfindungsgemäßer Polymere

Allgemeine Polymerisationsvorschrift zur Herstellung der erfindungsgemäßen Polymere nach dem Fällungsverfahren in tert.-Butanol

In einem 1-Liter Quickfitkolben mit Rückflusskühler, Gaseinleitung, Innenthermometer und Rührer werden 400 g tert.-Butanol vorgelegt und mit der berechneten Menge an 2-Acrylamido-2-methyl-propansulfonsäure (AMPS®, Lubrizol) versetzt. Anschließend wird durch $NH_3$-Einleitung neutralisiert (soll pH-Wert 6-7) und die berechnete Menge an Vernetzer und die berechneten Mengen der weiteren Comonomere in die Reaktionsmischung hinzugegeben. Sollte der

pH-Wert der Reaktionsmischung nach der Comonomerenzugabe in den sauren Bereich gedriftet sein, so wird dieser durch $NH_3$-Einleitung erneut neutralisiert (soll pH-Wert 6-7). Nach der Inertisierung der Mischung mit $N_2$ oder Argon wird bei einer Innentemperatur von 60 °C Dilauroylperoxid (DLP) oder Dimethyl 2,2'-azobis(2-methylpropionat) (V601) als Initiator zugesetzt und die Polymerisationsreaktion eingeleitet. Nach wenigen Minuten kommt es zum Ausfällen des fertigen Polymers. Die Mischung wird zwei Stunden auf Rückfluss erhitzt und das Polymer anschließend über eine Nutsche vom Lösemittel befreit und im Vakuum getrocknet. Diese Vorschrift ist allgemein auf alle im Weiteren beschriebenen Polymerisationsreaktionen anwendbar.

Zur Herstellung der Polymere der folgenden Beispiele 1 - 50 wurde nach der oben angegebenen allgemeinen Polymerisationsvorschrift vorgegangen. In den unter den folgenden Beispielen 1 - 50 aufgeführten Tabellen sind in den jeweiligen oberen Zeilen die Absolutmengen der zur Polymerisation eingesetzten Monomere und des Initiators (in Gramm "g") und in der jeweiligen mittleren Zeile die entsprechenden Mengen umgerechnet in Gew.-% angegeben. In der jeweiligen unteren Zeile ist der molare Anteil der Monomere (in mol-%) ohne Berücksichtigung des Initiators angegeben.

Die in den Beispielen angegebenen Abkürzungen der Monomere, Vernetzer und des Initiators haben folgende Bedeutung:

| | |
|---|---|
| AMP5 | = 2-Acrylamido-2-methyl-propansulfonsäure |
| HEEA | = Hydroxethylacrylamid |
| DMAAm | = Dimethylacrylamid |
| NIPA | = Isopropylacrylamid |
| NVP | = N-Vinylpyrrolidon |
| MAS | = Methacrylsäure |
| GPTA | = Glycerinpropoxylattriacrylat |
| $GP_{15}TA$ | = Glycerinpropoxylattriacrylat (15/3 PO/OH) |
| TMPTA-PO-3 | = Trimethylolpropanpropoxytriacrylat (3/3 PO/OH) |
| TMPTA-EO-3 | = Trimethylolpropanethoxytriacrylat (3/3 EO/OH) |
| TMPTA-EO-15 | = Trimethylolpropanethoxytriacrylat (15/3 EO/OH) |
| V601 | = Dimethyl 2,2'-azobis(2-methylpropionat) |
| DLP | = Dilauroylperoxid |

Beispiel 1:

| Menge | AMP5 | DMAAm | GPTA | V601 |
|---|---|---|---|---|
| g | 100,0 | 8,50 | 2,08 | 1,4 |
| Gew.-% | 89,3 | 7,5 | 1,9 | 1,3 |
| mol-% | 84,15 | 15,0 | 0,85 | - |

Beispiel 2:

| Menge | AMP5 | DMAAm | GPTA | V601 |
|---|---|---|---|---|
| g | 100,0 | 12,10 | 2,22 | 1,4 |
| Gew.-% | 86,4 | 10,5 | 1,9 | 1,2 |
| mol-% | 79,15 | 20,0 | 0,85 | - |

Beispiel 3:

| Menge | AMP5 | DMAAm | GPTA | V601 |
|---|---|---|---|---|
| g | 100,0 | 16,10 | 2,37 | 1,4 |
| Gew.-% | 83,4 | 13,4 | 2,0 | 1,2 |
| mol-% | 74,15 | 25,0 | 0,85 | - |

Beispiel 4:

| Menge | AMP5 | HEEA | GP15TA | V601 |
|---|---|---|---|---|
| g | 100,00 | 6,20 | 0,90 | 1,90 |
| Gew.-% | 91,7 | 5,7 | 0,9 | 1,7 |
| mol-% | 89,75 | 10,0 | 0,25 | - |

Beispiel 5:

| Menge | AMP5 | HEEA | GP15TA | V601 |
|---|---|---|---|---|
| g | 100,00 | 9,85 | 1,90 | 2,00 |
| Gew.-% | 87,9 | 8,7 | 1,7 | 1,8 |
| mol-% | 84,5 | 15,0 | 0,50 | - |

Beispiel 6:

| Menge | AMP5 | HEEA | GP15TA | V601 |
|---|---|---|---|---|
| g | 100,00 | 14,10 | 4,02 | 2,20 |
| Gew.-% | 83,1 | 11,7 | 3,3 | 1,8 |
| mol-% | 79,0 | 20,0 | 1,00 | - |

Beispiel 7:

| Menge | AMP5 | HEEA | GP15TA | V601 |
|---|---|---|---|---|
| g | 80,00 | 15,20 | 6,97 | 1,80 |
| Gew.-% | 76,9 | 14,7 | 6,7 | 1,7 |
| mol-% | 73,0 | 25,0 | 2,00 | - |

Beispiel 8:

| Menge | AMP5 | NIPA | TMPTA-PO-3 | DLP |
|---|---|---|---|---|
| g | 100,00 | 13,80 | 2,88 | 2,40 |
| Gew.-% | 84,0 | 11,6 | 2,4 | 2,0 |
| mol-% | 79,0 | 20,0 | 1,00 | - |

Beispiel 9:

| Menge | AMP5 | NIPA | TMPTA-EO-3 | DLP |
|---|---|---|---|---|
| g | 100,00 | 13,80 | 2,60 | 2,40 |
| Gew.-% | 84,2 | 11,6 | 2,2 | 2,0 |
| mol-% | 79,0 | 20,0 | 1,00 | - |

Beispiel 10:

| Menge | AMP5 | NIPA | TMPTA-EO-15 | DLP |
|---|---|---|---|---|
| g | 80,00 | 11,05 | 4,65 | 2,00 |
| Gew.-% | 81,9 | 11,3 | 4,8 | 2,0 |
| mol-% | 79,0 | 20,0 | 1,00 | - |

Beispiel 11:

| Menge | AMP5 | DMAAm | HEEA | GPTA | V601 |
|---|---|---|---|---|---|
| g | 100,00 | 6,05 | 7,05 | 1,95 | 2,10 |
| Gew.-% | 85,4 | 5,2 | 6,0 | 1,7 | 1,7 |
| mol-% | 79,2 | 10,0 | 10,05 | 0,75 | - |

Beispiel 12:

| Menge | AMP5 | DMAAm | HEEA | GPTA | V601 |
|---|---|---|---|---|---|
| g | 100,00 | 3,20 | 14,95 | 3,50 | 2,30 |
| Gew.-% | 80,7 | 2,6 | 12,1 | 2,7 | 1,9 |
| mol-% | 73,9 | 4,9 | 19,9 | 1,3 | - |

Beispiel 13:

| Menge | AMP5 | DMAAm | HEEA | GP15TA | V601 |
|---|---|---|---|---|---|
| g | 100,00 | 12,80 | 3,70 | 1,05 | 2,30 |
| Gew.-% | 83,4 | 10,7 | 3,1 | 0,9 | 1,9 |
| mol-% | 74,75 | 20,0 | 5,0 | 0,25 | - |

Beispiel 14:

| Menge | AMP5 | DMAAm | HEEA | GP15TA | V601 |
|---|---|---|---|---|---|
| g | 100,00 | 9,75 | 7,58 | 6,49 | 2,30 |
| Gew.-% | 79,3 | 7,7 | 6,0 | 5,2 | 1,8 |
| mol-% | 73,5 | 15,0 | 10,0 | 1,50 | - |

Beispiel 15:

| Menge | AMP5 | DMAAm | NVP | GPTA | V601 |
|---|---|---|---|---|---|
| g | 100,00 | 12,60 | 1,75 | 2,29 | 1,20 |
| Gew.-% | 84,9 | 10,7 | 1,5 | 1,9 | 1,9 |
| mol-% | 76,5 | 20,15 | 2,50 | 0,85 | - |

Beispiel 16:

| Menge | AMP5 | DMAAm | NVP | GPTA | V601 |
|---|---|---|---|---|---|
| g | 100,00 | 13,00 | 3,65 | 2,36 | 1,20 |
| Gew.-% | 83,2 | 10,8 | 3,0 | 2,0 | 1,0 |
| mol-% | 74,0 | 20,1 | 5,05 | 0,85 | - |

Beispiel 17:

| Menge | AMP5 | DMAAm | NVP | GPTA | V601 |
|---|---|---|---|---|---|
| g | 100,00 | 13,95 | 5,70 | 2,54 | 1,30 |
| Gew.-% | 81,0 | 11,3 | 4,6 | 2,1 | 1,1 |
| mol-% | 70,9 | 20,7 | 7,53 | 0,87 | - |

Beispiel 18:

| Menge | AMP5 | DMAAm | HEEA | NVP | GPTA | DLP |
|---|---|---|---|---|---|---|
| g | 100,00 | 9,39 | 3,63 | 1,75 | 2,29 | 2,50 |
| Gew.-% | 83,6 | 7,9 | 3,0 | 1,5 | 1,9 | 2,1 |
| mol-% | 76,55 | 15,1 | 5,0 | 2,50 | 0,85 | - |

Beispiel 19:

| Menge | AMP5 | DMAAm | HEEA | NVP | TMPTA-PO-3 | DLP |
|---|---|---|---|---|---|---|
| g | 100,00 | 3,00 | 3,51 | 6,80 | 2,88 | 2,40 |
| Gew.-% | 84,3 | 2,5 | 3,0 | 5,7 | 2,5 | 2,0 |
| mol-% | 79,0 | 5,0 | 5,0 | 10,0 | 1,00 | - |

Beispiel 20:

| Menge | AMP5 | DMAAm | HEEA | NVP | TMPTA-EO-3 | DLP |
|---|---|---|---|---|---|---|
| g | 100,00 | 6,00 | 3,60 | 3,50 | 5,30 | 2,40 |
| Gew.-% | 82,8 | 5,0 | 3,0 | 2,9 | 4,3 | 2,0 |
| mol-% | 78,0 | 9,8 | 5,1 | 5,10 | 2,00 | - |

Beispiel 21:

| Menge | AMP5 | DMAAm | MAS | GPTA | V601 |
|---|---|---|---|---|---|
| g | 100,00 | 12,60 | 0,27 | 2,29 | 1,50 |
| Gew.-% | 85,7 | 10,8 | 0,2 | 2,0 | 1,3 |
| mol-% | 78,0 | 20,6 | 0,55 | 0,85 | - |

Beispiel 22:

| Menge | AMP5 | DMAAm | MAS | GPTA | V601 |
|---|---|---|---|---|---|
| g | 100,00 | 12,60 | 1,35 | 2,29 | 1,40 |
| Gew.-% | 85,0 | 10,7 | 1,2 | 1,9 | 1,2 |
| mol-% | 76,5 | 20,15 | 2,5 | 0,85 | - |

Beispiel 23:

| Menge | AMP5 | DMAAm | MAS | GPTA | V601 |
|---|---|---|---|---|---|
| g | 100,00 | 13,00 | 2,80 | 2,36 | 1,50 |
| Gew.-% | 83,6 | 10,9 | 2,3 | 2,0 | 1,2 |
| mol-% | 74,0 | 20,15 | 5,0 | 0,85 | - |

Beispiel 24:

| Menge | AMP5 | DMAAm | MAS | GPTA | V601 |
|---|---|---|---|---|---|
| g | 100,00 | 13,80 | 4,40 | 2,47 | 1,60 |
| Gew.-% | 81,8 | 11,3 | 3,6 | 2,0 | 1,3 |
| mol-% | 71,1 | 20,5 | 7,55 | 0,85 | - |

Beispiel 25:

| Menge | AMP5 | DMAAm | MAS | GPTA | V601 |
|---|---|---|---|---|---|
| g | 100,00 | 13,95 | 6,00 | 2,54 | 1,70 |
| Gew.-% | 80,5 | 11,2 | 4,8 | 2,1 | 1,4 |
| mol-% | 69,1 | 20,1 | 9,95 | 0,85 | - |

Beispiel 26:

| Menge | AMP5 | DMAAm | MAS | GPTA | V601 |
|---|---|---|---|---|---|
| g | 100,00 | 14,90 | 9,60 | 2,54 | 1,70 |
| Gew.-% | 77,7 | 11,6 | 7,5 | 2,0 | 1,2 |
| mol-% | 64,3 | 20,0 | 14,9 | 0,8 | - |

Beispiel 27:

| Menge | AMP5 | DMAAm | MAS | NVP | GPTA | V601 |
|---|---|---|---|---|---|---|
| g | 100,00 | 13,20 | 2,85 | 0,75 | 2,39 | 1,20 |
| Gew.-% | 83,1 | 11,0 | 2,4 | 0,5 | 2,0 | 1,0 |
| mol-% | 73,0 | 20,05 | 5,0 | 1,0 | 0,84 | - |

Beispiel 28:

| Menge | AMP5 | DMAAm | MAS | NVP | TMPTA-PO-3 | V601 |
|---|---|---|---|---|---|---|
| g | 100,00 | 13,20 | 2,85 | 0,75 | 2,60 | 1,20 |
| Gew.-% | 82,9 | 10,9 | 2,4 | 0,6 | 2,2 | 1,0 |
| mol-% | 73,0 | 20,05 | 5,1 | 1,0 | 0,85 | - |

Beispiel 29:

| Menge | AMP5 | DMAAm | MAS | NVP | TMPTA-EO-3 | V601 |
|---|---|---|---|---|---|---|
| g | 100,00 | 13,20 | 2,85 | 0,75 | 2,40 | 1,20 |
| Gew.-% | 83,1 | 11,0 | 2,4 | 0,5 | 2,0 | 1,0 |
| mol-% | 73,0 | 20,05 | 5,1 | 1,0 | 0,85 | - |

Beispiel 30:

| Menge | AMP5 | DMAAm | MAS | NVP | GP15TA | V601 |
|---|---|---|---|---|---|---|
| g | 100,00 | 13,20 | 2,85 | 0,75 | 3,70 | 1,20 |
| Gew.-% | 82,2 | 10,8 | 2,3 | 0,6 | 3,1 | 1,0 |
| mol-% | 73,0 | 20,05 | 5,1 | 1,0 | 0,85 | - |

Beispiel 31:

| Menge | AMP5 | DMAAm | HEEA | MAS | GPTA | V601 |
|---|---|---|---|---|---|---|
| g | 100,00 | 2,85 | 3,30 | 2,49 | 2,45 | 1,40 |
| Gew.-% | 88,9 | 2,5 | 2,9 | 2,2 | 2,3 | 1,2 |
| mol-% | 84,0 | 5,0 | 5,0 | 5,0 | 1,00 | - |

Beispiel 32:

| Menge | AMP5 | DMAAm | HEEA | MAS | GP15TA | V601 |
|---|---|---|---|---|---|---|
| g | 100,00 | 6,45 | 7,50 | 2,85 | 4,30 | 1,50 |
| Gew.-% | 81,6 | 5,3 | 6,1 | 2,3 | 3,5 | 1,2 |
| mol-% | 74,0 | 10,0 | 10,0 | 5,0 | 1,00 | - |

Beispiel 33:

| Menge | AMP5 | DMAAm | HEEA | MAS | TMPTA-EO-3 | V601 |
|---|---|---|---|---|---|---|
| g | 100,00 | 3,42 | 16,00 | 3,00 | 3,00 | 1,60 |
| Gew.-% | 78,7 | 2,7 | 12,6 | 2,4 | 2,4 | 1,2 |
| mol-% | 69,1 | 4,9 | 20 | 5,0 | 1,00 | - |

Beispiel 34:

| Menge | AMP5 | DMAAm | HEEA | MAS | TMPTA-PO-3 | V601 |
|---|---|---|---|---|---|---|
| g | 100,00 | 9,66 | 3,75 | 2,80 | 3,05 | 1,50 |
| Gew.-% | 82,8 | 8,0 | 3,2 | 2,3 | 2,5 | 1,2 |
| mol-% | 74,0 | 15,0 | 5,0 | 5,0 | 1,00 | - |

Beispiel 35:

| Menge | AMP5 | DMAAm | HEEA | MAS | TMPTA-PO-3 | V601 |
|---|---|---|---|---|---|---|
| g | 100,00 | 9,66 | 3,75 | 2,80 | 6,20 | 1,50 |
| Gew.-% | 80,7 | 7,8 | 3,0 | 2,3 | 5,0 | 1,2 |
| mol-% | 73,3 | 14,8 | 4,9 | 4,9 | 2,00 | 1,0- |

Beispiel 36:

| Menge | AMP5 | HEEA | GPTA | V601 |
|---|---|---|---|---|
| g | 95,00 | 13,30 | 1,23 | 1,40 |
| Gew.-% | 85,6 | 12,0 | 1,1 | 1,3 |
| mol-% | 79,5 | 20,0 | 0,50 | - |

Beispiel 37:

| Menge | AMP5 | HEEA | GPTA | V601 |
|---|---|---|---|---|
| g | 95,00 | 13,30 | 1,85 | 1,40 |
| Gew.-% | 85,2 | 11,9 | 1,7 | 1,3 |
| mol-% | 79,3 | 20,0 | 0,75 | - |

Beispiel 38:

| Menge | AMP5 | HEEA | GPTA | V601 |
|---|---|---|---|---|
| g | 95,00 | 13,40 | 2,49 | 1,40 |
| Gew.-% | 84,6 | 12,0 | 2,2 | 1,2 |
| mol-% | 78,9 | 20,1 | 1,00 | - |

Beispiel 39:

| Menge | AMP5 | DMAAm | GPTA | V601 |
|---|---|---|---|---|
| g | 95,00 | 11,40 | 1,04 | 1,30 |
| Gew.-% | 87,3 | 10,5 | 1,0 | 1,2 |
| mol-% | 79,6 | 19,98 | 0,42 | - |

Beispiel 40:

| Menge | AMP5 | DMAAm | GPTA | V601 |
|---|---|---|---|---|
| g | 95,00 | 11,40 | 1,39 | 1,30 |
| Gew.-% | 87,1 | 10,5 | 1,3 | 1,2 |
| mol-% | 79,5 | 19,95 | 0,56 | - |

Beispiel 41:

| Menge | AMP5 | DMAAm | GPTA | V601 |
|---|---|---|---|---|
| g | 95,00 | 11,40 | 1,74 | 1,30 |
| Gew.-% | 86,8 | 10,4 | 1,6 | 1,2 |
| mol-% | 79,4 | 19,9 | 0,70 | - |

Beispiel 42:

| Menge | AMP5 | DMAAm | GPTA | V601 |
|---|---|---|---|---|
| g | 95,00 | 11,40 | 2,81 | 1,30 |
| Gew.-% | 86,0 | 10,3 | 2,5 | 1,2 |
| mol-% | 79,1 | 19,8 | 1,1 | - |

Beispiel 43:

| Menge | AMP5 | DMAAm | GPTA | V601 |
|---|---|---|---|---|
| g | 95,00 | 8,10 | 0,98 | 1,30 |
| Gew.-% | 90,1 | 7,7 | 0,9 | 1,2 |
| mol-% | 84,5 | 15,1 | 0,4 | - |

Beispiel 44:

| Menge | AMP5 | DMAAm | GPTA | V601 |
|---|---|---|---|---|
| g | 95,00 | 8,15 | 1,64 | 1,30 |
| Gew.-% | 89,5 | 7,8 | 1,5 | 1,2 |
| mol-% | 84,2 | 15,10 | 0,70 | - |

Beispiel 45:

| Menge | AMP5 | DMAAm | GPTA | V601 |
|---|---|---|---|---|
| g | 95,00 | 8,20 | 2,31 | 1,30 |
| Gew.-% | 88,9 | 7,7 | 2,2 | 1,2 |
| mol-% | 83,9 | 15,1 | 1,0 | - |

Beispiel 46:

| Menge | AMP5 | DMAAm | GPTA | V601 |
|---|---|---|---|---|
| g | 95,00 | 5,15 | 0,93 | 1,20 |
| Gew.-% | 92,9 | 5,0 | 0,9 | 1,2 |
| mol-% | 89,4 | 10,2 | 0,4 | - |

Beispiel 47:

| Menge | AMP5 | DMAAm | GPTA | V601 |
|---|---|---|---|---|
| g | 95,00 | 5,15 | 1,55 | 1,20 |
| Gew.-% | 92,3 | 5,0 | 1,5 | 1,2 |
| mol-% | 89,2 | 10,1 | 0,70 | - |

Beispiel 48:

| Menge | AMP5 | DMAAm | GPTA | V601 |
|---|---|---|---|---|
| g | 95,00 | 5,15 | 2,18 | 1,20 |
| Gew.-% | 91,7 | 5,0 | 2,1 | 1,2 |
| mol-% | 88,9 | 10,1 | 1,0 | - |

Beispiel 49:

| Menge | AMP5 | DMAAm | GPTA | V601 |
|---|---|---|---|---|
| g | 100,00 | 2,55 | 1,55 | 1,20 |
| Gew.-% | 95,0 | 2,4 | 1,5 | 1,1 |
| mol-% | 94,3 | 5,0 | 0,7 | - |

Beispiel 50:

| Menge | AMP5 | DMAAm | GPTA | V601 |
|---|---|---|---|---|
| g | 100,00 | 2,55 | 2,18 | 1,20 |
| Gew.-% | 94,4 | 2,4 | 2,1 | 1,1 |
| mol-% | 94,0 | 5,01 | 0,99 | - |

B) Beispiele zur Verdickung in Gegenwart von Salz und zur Sensorik erfindungsgemäßer Polymere

B1) Verdickung in Gegenwart von Salz

Erfindungsgemäße Polymere bewirken eine höhere Viskosität in Gegenwart von Salz als das Polymer Aristoflex® AVC der Firma Clariant aus dem Stand der Technik. Die Viskositäten wurden bei einer Polymerkonzentration von 2 Gew.-% in einer Lösung von 2 Gew.-% Natriumchlorid in Wasser ermittelt.

Die Viskositäten wurden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 pa·s, Spindel 2 für Viskositäten von maximal 1 000 mPa · s, Spindel 3 für Viskositäten von maximal 5 000 mPa · s, Spindel 4 für Viskositäten von maximal 10 000 mPa · s, Spindel 5 für Viskositäten von maximal 20 000 mPa · s, Spindel 6 für Viskositäten von maximal 50 000 mPa · s und Spindel 7 für Viskositäten von maximal 200 000 mPa · s gewählt.

| Polymer | Viskosität (2 Gew.-% Polymer in einer Lösung von 2 Gew.-% NaCl in Wasser) [mPa · s] |
|---|---|
| Aristoflex® AVC (Vergleich) | 3680 |
| Beispiel 36 | 6740 |
| Beispiel 37 | 7940 |
| Beispiel 38 | 5800 |
| Beispiel 39 | 5230 |
| Beispiel 40 | 4600 |
| Beispiel 41 | 5540 |
| Beispiel 2 | 4760 |

(fortgesetzt)

| Polymer | Viskosität (2 Gew.-% Polymer in einer Lösung von 2 Gew.-% NaCl in Wasser) [mPa · s] |
|---|---|
| Beispiel 42 | 4160 |
| Beispiel 43 | 3890 |
| Beispiel 44 | 7160 |
| Beispiel 45 | 6320 |
| Beispiel 46 | 3810 |
| Beispiel 47 | 6140 |
| Beispiel 48 | 7360 |
| Beispiel 49 | 6000 |
| Beispiel 50 | 5500 |
| Beispiel 15 | 5480 |
| Beispiel 23 | 5600 |
| Beispiel 27 | 6700 |

B2) Sensorik

Zusätzlich wurden Sensoriktests innerhalb eines Paneltests mit 10 Probanden durchgeführt, wobei die Sensorik der erfindungsgemäßen Polymere unter Verwendung der nachfolgenden Basisformulierung geprüft wurde:

| Inhaltsstoff | Gew.-% |
|---|---|
| Myritol 318 | 3 |
| Cetiol MM | 2,5 |
| Lanette O | 2 |
| Imwitor 370P | 1 |
| Eutanol G | 1 |
| Polymer | 0,4 |
| Wasser | ad 100 |
| Glycerin | 7,5 |
| Ethanol | 3 |
| Tocopherylacetate | 1 |
| Aloe Barbadensis | 1 |
| NaOH (10 Gew.-% in Wasser) | 0,2 |
| Phenonip | 0,6 |

Hierbei wurden Formulierungen unter Verwendung der im Folgenden genannten Polymere hergestellt, wobei Aristoflex® AVC (ein Polymer entsprechend EP 1 116 733 auf Basis von 2-Acrylamido-2-methyl-propansulfonsäure, Vinylpyrrolidon und Trimethylolpropantriacrylat als Vernetzer) als Vergleichspolymer verwendet wurde, und sensorisch bewertet. Die Gesamtbewertung der Polymere wurde mit dem Schlüssel ++ = sehr gut, + = gut, o = befriedigend, - = unbefriedigend bewertet.

| Polymer | Sensorik | Gesamturteil |
|---|---|---|
| Aristoflex® AVC (Vergleich) | zieht schnell ein, kurz pflegend | - |

(fortgesetzt)

| Polymer | Sensorik | Gesamturteil |
|---|---|---|
| Beispiel 39 | zieht langsam ein, Film liegt auf der Haut, pflegend | ○ |
| Beispiel 40 | zieht langsam ein, Film liegt auf der Haut, pflegend | ○ |
| Beispiel 41 | zieht langsam ein, lange pflegend | + |
| Beispiel 2 | Zieht langsam ein, sehr lange pflegend | ++ |
| Beispiel 42 | Zieht langsam ein, sehr lange pflegend | ++ |
| Beispiel 36 | Zieht langsam ein, lange pflegend | + |
| Beispiel 37 | zieht langsam ein, sehr lange pflegend | ++ |
| Beispiel 38 | zieht langsam ein, sehr lange pflegend | ++ |
| Beispiel 43 | zieht langsam ein, lange pflegend | + |
| Beispiel 44 | Zieht langsam ein, sehr lange pflegend | ++ |
| Beispiel 45 | Zieht langsam ein, sehr lange pflegend | ++ |
| Beispiel 46 | Zieht sehr langsam ein, pflegend | ○ |
| Beispiel 47 | Zieht sehr langsam ein, pflegend | ○ |
| Beispiel 48 | Zieht sehr langsam ein, pflegend | ○ |
| Beispiel 49 | Zieht langsam ein, pflegend | ○ |
| Beispiel 50 | Zieht langsam ein, pflegend | ○ |
| Beispiel 15 | zieht langsam ein, sehr lange pflegend | ++ |
| Beispiel 23 | zieht langsamer ein, Film liegt auf der Haut, reichhaltiger, lange pflegend | ++ |
| Beispiel 27 | zieht langsam ein, pflegend | ○ |

C) Beispiele zu erfindungsgemäßen kosmetischen Formulierungen

Bei allen folgenden Prozentangaben in den Beispielen handelt es sich um Gewichtsprozent (Gew.-%), sofern nicht explizit anders angegeben.

Folgende kosmetische Formulierungen wurden mit erfindungsgemäßen Polymeren hergestellt:

Formulierungbeispiele 1 - 8: Handsanitizerformulierungen

| Formulierung Nr. | 1 | 2 | 3 | 4 | 5 | 6 | 7 | 8 |
|---|---|---|---|---|---|---|---|---|
| Inhaltsstoff | Menge des jeweiligen Inhaltsstoffs [Gew.-%] | | | | | | | |
| Polymer nach Bsp. 2 | 1,0 | - | 1,2 | - | 0,8 | - | 1,0 | - |
| Polymer nach Bsp. 27 | - | 1,0 | - | 1,2 | - | 1,0 | - | 1,0 |
| Ethanol (96 Gew.-% in Wasser) | 70,0 | 40,0 | - | - | - | 40,0 | 70,0 | 50,0 |
| Isopropanol | - | - | 40,0 | 70,0 | 70,0 | - | - | - |
| Piroctone Olamine | - | - | - | - | 1,0 | 0,5 | - | 1,0 |
| Triclosan | - | - | - | - | - | - | 0,5 | - |
| Wasser | 29,0 | 59,0 | 58,8 | 28,8 | 28,2 | 58,5 | 28,5 | 48,0 |

Herstellung:

Das Polymer wird in Wasser eingerührt, der entsprechende Alkohol, in dem antibakterielle Wirkstoffe wie Piroctone Olamine oder Triclosan gelöst sein können, zugegeben und homogenisiert. Es resultiert ein klares Gel.

Formulierungsbeispiele 9 - 12: Haarpflegegele für starken Halt und und exzellentes Styling

| Formulierung Nr. | 9 | 10 | 11 | 12 |
|---|---|---|---|---|
| Inhaltsstoff | Menge des jeweiligen Inhaltsstoffs [Gew.-%] | | | |
| Polymer nach Beispiel 6 | 1,0 | - | 1,0 | - |
| Polymer nach Beispiel 23 | - | 1,0 | - | 1,0 |
| Sorbitol | 0,5 | 0,5 | - | - |
| Wasser | ad 100 | ad 100 | ad 100 | ad 100 |
| Carbomer | - | 0,5 | 0,5 | - |
| NaOH | - | q.s. | q.s. | - |
| PEG-40 Hydrogenated Castor Oil | 1,0 | 1,0 - | 1,0 | - |
| Fragrance | 0,3 | 0,3 | - | 0,3 |
| Ethanol (96 Gew.-% in Wasser) | 10,0 | 10,0 | 5,0 | - |
| Diaformer Z-712 N (Acrylates/Lauryl acrylates/Stearyl Acrylate/Ethylamine Oxide Methacrylate) | 4,5 | 4,5 | - | 6,0 |
| Luviskol VA 64 (PVP/VA) | 3,0 | 3,0 | 5,0 | - |
| Propylene Glycol | 1,0 | 1,0 | - | 1,0 |
| Panthenol | 0.5 | 0,5 | - | - |
| Dyestuff solution | q.s. | q.s. | q.s. | - |
| Phenoxyethanol | 1,0 | 1,0 | 0,5 | 0,7 |

Herstellung:

Die Polymere nach Beispiel 1 und 2 werden in Wasser (und gegebenenfalls Sorbitol) gelöst. Im Falle von Carbomerzugabe wird anschließend mit NaOH auf pH = 7 neutralisiert. Die übrigen Komponenten werden gegebenenfalls mit PEG-40 hydrogenated Castor Oil gemischt und in die verdickte Wasserphase eingerührt.

Formulierungsbeispiel 13: Oxidative Haarfärbeformulierung Färbebasis:

| Inhaltsstoff | Gew.-% |
|---|---|
| Polyquaternium-29 (Dihydroxyproyl Chitosan Trimonium Chloride) | 0,5 |
| m-Phenylendiamin | 0,08 |
| p-Phenylendiamine HCl | 0,30 |
| Resorcinol | 0,25 |
| Sodium Bisulfite | 0,30 |
| Sodium Laureth Sulfate | 3,50 |

(fortgesetzt)

| Inhaltsstoff | Gew.-% |
|---|---|
| Cetyl Alcohol | 15,00 |
| Ammonia (25 Gew.-%ig, wässrig) | 2,00 |
| Wasser | ad 100 |

Herstellung:

Cetyl alcohol und Sodium Laureth Sulfate werden auf 60°C erhitzt, gemischt und unter Rühren in die Wasserphase eingetragen, in der die übrigen Inhaltsstoffe gelöst wurden.

Entwicklergel:

| Inhaltsstoff | Gew.-% |
|---|---|
| Polymer nach Beispiel 6 | 1,5 |
| Wasserstoffperoxid (35 Gew.-%ig, wässrig) | 18 |
| Natriumpyrophosphat | 0,02 |
| 4-Methyl-6-(cyclohexyl)-1-hydroxy-2-pyridon | 0,002 |
| Propylenglykol | 1 |
| Wasser | ad 100 |

Herstellung:

4-Methyl-6-(cyclohexyl)-1-hydroxy-2-pyridon wird in Propylenglykol gelöst. Das erfindungsgemäße Polymer wird in Wasser aufgelöst, sodann wird die Wasserstoffperoxidlösung eingerührt, gefolgt vom Stabilisator Natriumpyrophosphat sowie der Propylenglykollösung.

Es entsteht ein Gel mit einer Viskosität von ca. 3 000 mPa · s bei 20 °C.

Färbeprozedur:

50 ml der Färbebasis werden mit 50 ml des Entwicklergels verrührt und auf das Haar appliziert. Nach 30 Minuten wird ausgespült.

Formulierungsbeispiel 14: Emulgatorfreies Haarbleichgel

| Inhaltsstoff | Gew.-% |
|---|---|
| Polymer nach Beispiel 27 | 1,0 |
| Wasserstoffperoxid (35 Gew.-%ig, wässrig) | 17 |
| Natriumpyrophosphat | 0,02 |
| Natriumstannat | 0,04 |
| 4-Methyl-6-(2,4,4-trimethylpentyl)-1-hydroxy-2-pyridon | 0,001 |
| Propylenglykol | 1 |
| Wasser | ad 100 |

Herstellung:

4-Methyl-6-(2,4,4-trimethylpentyl)-1-hydroxy-2-pyridon wird in Propylenglykol gelöst. Das Polymer wird in Wasser aufgelöst, sodann wird die Wasserstoffperoxidlösung eingerührt, gefolgt von den beiden Stabilisatoren und der Propylenglykollösung.

Formulierungsbeispiel 15: Fixiergel für Dauerwellen

| Inhaltsstoff | Gew.-% |
|---|---|
| Polymer nach Beispiel 23 | 0,8 |
| Wasserstoffperoxid (35 Gew.-%ig, wässrig) | 5 |
| Natriumpyrophosphat | 0,02 |
| 4-Methyl-6-(2,4,4-trimethylpentyl)-1-hydroxy-2-pyridon | 0,002 |
| Propylenglykol | 1 |
| Wasser | ad 100 |

Herstellung:

4-Methyl-6-(2,4,4-trimethylpentyl)-1-hydroxy-2-pyridon wird in Propylenglykol gelöst.

Das Polymer wird in Wasser gelöst, die Wasserstoffperoxidlösung und das Pyrophosphat sowie die Propylenglykollösung eingetragen und homogenisiert.

Formulierungsbeispiel 16: O/W Exfoliating Creme mit hohem Elektrolytgehalt (Na-Glykolat)

| Phase | Inhaltsstoff | Gew.-% |
|---|---|---|
| A | PEG-120 Methyl Glucose Dioleat | 1,5 |
| B | Wasser | ad 100 |
| C | Mineral Oil<br>Caprylyl Trimethicone | 5,0<br>3,0 |
| D | Polymer nach Beispiel 2 | 1,2 |
| E | Glykolsäure 30 Gew.-% in Wasser (neutralisiert mit NaOH zu pH = 4)<br>Konservierungsmittel | 6,0<br>q.s. |
| F | Laureth-7 | 3,0 |

Herstellung:

A unter Erwärmen in Phase B lösen. Phase C in Phase D dispergieren und in die Wasserphase einrühren. Anschließend Phasen E und F einrühren.

Formulierungsbeispiel 17: W/O Pflege-Hautmilch

| Phase | Inhaltsstoff | Gew.-% |
|---|---|---|
| A | Amodimethicone Glycerocarbamate<br>Cyclopentasiloxane<br>Paraffin Oil<br>Apricot Kernel Oil<br>Grape Seed Oil<br>Microcrystalline Wax<br>Stearic acid<br>Ethylhexyl Cocoate | 2,0<br>5,0<br>3,5<br>1,0<br>0,5<br>0,7<br>0,5<br>7,0 |
| B | Polymer nach Beispiel 27 | 0,3 |
| C | Wasser<br>Glycerin<br>Konservierungsmittel | ad 100<br>3,5<br>q.s. |

Herstellung:

Die Ölphase A auf 80 °C erhitzen und das Polymer B einrühren. Phase C langsam in kleinen Portionen unter starkem Rühren zugeben und auf Raumtemperatur abkühlen lassen.

Formulierungsbeispiel 18: Makeup-Remover mit exzellenten Hautgefühl

| Phase | Inhaltsstoff | Gew.-% |
|---|---|---|
| A | Isopropyl C12-15 Pareth-9 carboxylate | 5,0 |
| B | Sodium Cocoyl Glutamate (25 Gew.-%ige Lösung in Wasser)<br>Cocamidopropyl Betaine (30 Gew.-%ige Lösg. in Wasser)<br>Laureth-7<br>Wasser<br>Allantoin<br>Polypropylene Terephthalate<br>1,6 Hexanediol<br>Propylene Glycol<br>PEG-8<br>Panthenol<br>Poloxamer 407<br>Preservative<br>Polymer nach Beispiel 6 | 2,3<br>3,0<br>2,0<br>ad 100<br>0,3<br>1,0<br>2,0<br>2,0<br>2,0<br>0,5<br>3,0<br>q.s.<br>1,0 |

Herstellung:

Die Komponenten von B nacheinander in A lösen

Formulierungsbeispiel 19: Shampoo/Duschbad mit suspendierten Partikeln

| Phase | Inhaltsstoff | Gew.-% |
|---|---|---|
| A | Wasser | ad 100 |
| B | Polymer nach Beispiel 2 | 2,0 |

(fortgesetzt)

| Phase | Inhaltsstoff | Gew.-% |
|---|---|---|
| C | Sodium Laureth Sulfate (30 Gew.-% in Wasser)<br>Parfüm<br>Konservierungsmittel | 18,5<br>0,5<br>q.s. |
| D | Sodium Cocoyl Glutamate (25 Gew.-%ige Lösung in Wasser) | 20,0 |
| E | Synthetic Wax | 0,2 |

Herstellung:

Polymer in Wasser lösen, dann nacheinander Phasen C, D und E eintragen und homogenisieren.

Formulierungsbeispiel 20: Klares Deodorierendes Gel

| Phase | Inhaltsstoff | Gew.-% |
|---|---|---|
| A | PEG-40 hydrogenated Castor Oil<br>Parfum | 1,0<br>0,1 |
| B | Ethanol (96 Gew.-% in Wasser)<br>Piroctone Olamine (Octopirox®, Clariant) | 25,0<br>0,1 |
| C | Propylene Glycol<br>Diisopropyl Adipate<br>Wasser<br>Benzyl alcohol, Methylparaben, Propylparaben | 20,0<br>1,0<br>ad 100<br>0,2 |
| D | Polymer nach Beispiel 23 | 1,3 |
| E | Zitronensäure | q.s. |

Herstellung:

Phase A wird gemischt, sodann werden Phase B und Phase C nacheinander zugegeben und der pH-Wert mit Phase E auf pH = 5,5. angepasst. Abschießend wird Phase D eingerührt, bis ein homogenese klares Gel entsteht.

Formulierungsbeispiel 21: Mattierendes Serum

| Phase | Inhaltsstoff | Gew.-% |
|---|---|---|
| A | Wasser | 100 |
| B | Glycerin<br>Polymer nach Beispiel 23<br>Caprylyl Methicone<br>Cyclomethicone und Dimethicone Crosspolymer (Dow Corning 9040 Silicone Elastomer blend)<br>Fragrance<br>Preservative | 3,0<br>0,5<br>1,5<br>1,0<br>0,15<br>q.s. |

Herstellung:

Die Komponenten von B werden nacheinander in Phase A eingerührt.

Formulierungsbeispiel 22: Skin Whitening Gel

| Phase | Inhaltsstoff | Gew.-% |
|---|---|---|
| A | Allantoin | 0,5 |
| B | Wasser | ad 100 |
| C | Xanthan Gum | 0,5 |
| D | Ascorbic Acid 2-Glucoside | 2,0 |
| E | NaOH (25 Gew-%ige Lösung in Wasser) | q.s. |
| F | Glycerin<br>Ethanol (96 Gew.-% in Wasser)<br>PEG/PPG-18/18 Dimethicone (Dow Corning® 190, Dow Corning)<br>PEG-40 hydrogenated Castor Oil | 10,0<br>10,0<br>1,0<br>0,8 |
| G | Polymer nach Beispiel 27 | 1,0 |
| H | NaOH (25 Gew-%ige Lösung in Wasser) | q.s. |
| I | DMDM Hydantoin | q.s |

Herstellung:

Phase A wird in Phase B unter Erwärmen gelöst, Phase C eingerührt, Phase D zugegeben und mit Phase E auf pH = 6,5 eingestellt. Phase F wird gemischt und dann zugeben, anschließend wird Phase G zugegeben und gerührt, bis ein homogenes Gel erzielt wird. Mit Phase H wird der pH gegebenenfalls auf 6,5 eingestellt und das Konservierungsmittel I eingerührt.

Formulierungsbeispiel 23: Elegante O/W Hautpflege-Bodylotion mit geringer Klebrigkeit

| Phase | Inhaltsstoff | Gew.-% |
|---|---|---|
| A | Caprylic/Capric Triglyceride<br>Myristyl Myristate<br>Cetearyl Alcohol<br>Glyceryl Stearate Citrate<br>Octyldodecanol | 3,5<br>2,5<br>2,0<br>1,0<br>1,0 |
| B | Polymer nach Beispiel 17 | 0,6 |
| C | Wasser<br>Glycerin | ad 100<br>7,5 |
| D | Ethanol (96 Gew.-% in Wasser)<br>Dimethicone<br>Tocopheryl Acetate<br>Aloe Barbadensis<br>Preservative<br>Fragrance | 3,0<br>3,0<br>1,0<br>1,0<br>q.s.<br>q.s. |
| E | NaOH (10 Gew-% in Wasser) | q.s. |

Herstellung:

Phase A wird bei 70 °C aufgeschmolzen, Phase B eingestreut und die auf 70 °C erwärmte Phase C eingerührt. Nach Abkühlen auf 35 °C wird Pahse D eingerührt und abschließend mit Phase E auf pH = 6 eingestellt.

Formulierungsbeispiel 24: Tensidfreie Anti-Ageing O/W Gelcreme mit hautfaltenreduzierender Funktion

| Phase | Inhaltsstoff | Gew.-% |
|---|---|---|
| A | Dicaprylyl Ether<br>Caprylic/Capric Triglyceride<br>Cetearyl Alcohol<br>Preservative | 5,0<br>5,0<br>2,0<br>q.s. |
| B | Ubiquinone | 0,1 |
| C | Polymer nach Beispiel 23 | 1,1 |
| D | Sodium Hyaluronate (Dekluron)<br>Glycerin | 0,3<br>8,0 |
| E | Wasser<br>Mica und Titanium Dioxide und Tin Oxide (Prestige® Soft Orange, Eckart) | ad 100<br>0,5 |
| F | Tocopheryl Acetate | 0,3 |
| G | NaOH (10 Gew.-% in Wasser) | q.s |

Herstellung:

Phase A wird bei 80°C geschmolzen, Phase B und Phase C nacheinander eingerührt. Phase D wird in Phase E vorgelöst und zugegeben. Phase F wird bei 35 °C eingerührt und mit Phase G der pH-Wert auf 6,0 eingestellt. Es entsteht eine Gelcreme.

Formulierungsbeispiel 25: Tensidfreie Anti-Ageing O/W Gelcreme

| Phase | Inhaltsstoff | Gew.-% |
|---|---|---|
| A | Dicaprylyl Ether<br>Caprylic/Capric Triglyceride<br>Cetearyl Alcohol<br>Preservative | 5,0<br>5,0<br>2,0<br>q.s. |
| B | Ubiquinone | 0,1 |
| C | Polymer nach Beispiel 27 | 1,1 |
| D | Xanthan Gum<br>Glycerin | 0,2<br>8,0 |
| E | Wasser<br>Mica und Titanium Dioxide und Tin Oxide (Prestige® Soft Orange, Eckart) | ad 100<br>0,5 |
| F | Tocopheryl Acetate | 0,3 |
| G | NaOH (10 Gew.-% in Wasser) | q.s |

Herstellung:

Phase A wird bei 80 °C geschmolzen, Phase B und Phase C nacheinander eingerührt. Phase D wird in Phase E vorgelöst und zugegeben. Phase F wird bei 35 °C eingerührt und mit Phase G der pH-Wert auf 6,0 eingestellt. Es entsteht eine Gelcreme.

Formulierungsbeispiel 26: O/W Selbstbräunungscreme mit Moisturizing-Effekt

| Phase | Inhaltsstoff | Gew.-% |
|---|---|---|
| A | Cetyl Phsophate<br>Glyceryl Stearate<br>Cetearyl Alcohol<br>Isohexadecane<br>Isopropyl Palmitate<br>Caprylyl Methicone | 1,0<br>0,5<br>0,5<br>8,0<br>7,0<br>1,0 |
| B | Polymer nach Beispiel 6 | 1,0 |
| C | Wasser<br>Sodium Cocoyl Glutamate<br>Glycerin<br>NaOH (10 Gew.-% in Wasser) | ad 100<br>0,5<br>5,0<br>0,5 |
| D | Tocopheryl Acetate<br>Fragrance<br>Preservative | 1,0<br>0,2<br>q.s. |
| E | Dihydroxyacetone<br>Wasser | 5,0<br>8,0 |

Herstellung:

Phase A wird bei 80 °C geschmolzen, Phase B und Phase C nacheinander eingerührt. Phase D wird bei 30 °C zugeben und Phase E abschließend eingerührt. Es resultiert eine Creme mit einen pH-Wert von 4,2.

Formulierungsbeispiel 27 - 32: W/O Sonnenschutzformulierungen mit hohem Schutzfaktor

In Sonnenschutzmitteln tragen die erfindungsgemäßen Polymere zur besseren Verteilbarkeit der Sonnenschutzformulierung bei und verleihen dem kosmetischen und pharmazeutischen Produkt ein angenehmes Hautgefühl und gutes Spreitungsvermögen.

Die in der folgenden Tabelle gezeigten Sonnenschutzformulierungen wurden hergestellt.

| Formulierung Nr. | 27 | 28 | 29 | 30 | 31 | 32 |
|---|---|---|---|---|---|---|
| Inhaltsstoff | Menge des jeweiligen Inhaltsstoffs [Gew.-%] | | | | | |
| $C_{12-15}$ Alkyl benzoate | 8 | 8 | 8 | 8 | 8 | 8 |
| Caprylic Capric Triglyceride | 5 | 5 | 5 | 5 | 5 | 5 |
| Octocrylene | 9 | - | 5 | 4 | - | - |
| Ethylhexyl Methoxycinnamate | 7 | 7 | 7 | - | 6 | 6 |
| Butyl Methoxydibenzoylmethane | 2,5 | - | 2,5 | - | - | - |
| Disodium Phenyl Dibenzimidazole Tetrasulfonate | - | - | - | - | - | 3 |
| Ethylhexyl Bis-Isopentylbezoxazolyl-phenyl-melamine | - | - | - | - | 2 | - |

(fortgesetzt)

| Formulierung Nr. | 27 | 28 | 29 | 30 | 31 | 32 |
|---|---|---|---|---|---|---|
| Inhaltsstoff | Menge des jeweiligen Inhaltsstoffs [Gew.-%] | | | | | |
| Diethylamino Hydroxybenzoyl Hexyl Benzoate | - | - | 2 | 1 | - | - |
| Bis Ethylhexyloxyphenol Methoxyphenyl Triazine | - | 3 | - | 2 | 4 | 3 |
| Methylene Bis-Benzotriazolyl Tetramethylbutylphenol | - | 3 | - | - | - | 2 |
| Ethylhexyl Triazone | - | - | - | 3 | - | - |
| Diethylhexyl Butamido Triazone | - | - | - | - | 2 | - |
| Polysilicone-15 | - | - | 2 | - | - | - |
| Phenylbenzimidazole Sulfonic Acid | - | - | - | 3 | - | - |
| Titanium Dioxide | - | 5 | 3 | 4 | 5 | 5 |
| Cetearyl Alcohol | 1 | 1 | 1 | 1 | 1 | 1 |
| Sunflower Seed Oil Sorbitol Esters | 2 | 2 | 2 | 2 | 2 | 2 |
| Preservative | q.s. | q.s. | q.s. | q.s. | q.s. | q.s. |
| Kaliumcetylphosphat | 3 | 3 | 3 | 3 | 3 | 3 |
| Polymer nach Beispiel 4 | 1 | 0,6 | - | - | - | - |
| Polymer nach Beispiel 23 | - | - | 0,5 | 0,9 | - | - |
| Polymer nach Beispiel 27 | - | - | - | - | 1 | 1 |
| Wasser | ad 100 | ad 100 | ad 100 | ad 100 | ad 100 | ad 100 |
| Nylon | - | 0,5 | - | - | - | - |
| Bis-Ethylhexyl Hydroxydimethoxy Benzylmalonate | - | - | 1 | - | - | - |
| Talc | - | - | - | - | 0,5 | - |

Herstellung:

Zur Herstellung wurden die öllöslichen Komponenten auf 80°C erhitzt, Kaliumcetylphosphat sowie das erfindungsgemäße Polymer nacheinander eingestreut und die vereinigten wasserlöslichen Phasen langsam unter starkem Rühren in die Ölphase eingetragen. Die gebildeten Emulsionen wurden unter Rühren auf Raumtemperatur abkühlen lassen.

Die in den Formulierungsbeispielen 27-32 verwendeten Sonnenschutzfilter, ihre Markennamen sowie ihr UV-Schutzbereich sind in der folgenden Tabelle aufgeführt.

| Sonnenschutzfilter | Markenname | Schutzbereich (UV-A/UV-B) |
|---|---|---|
| Octocylene | Neo Heliopan® 303 | B |
| Ethylhexyl Methoxycinnamate | Neo Heliopan® AV | B |
| Butyl Methoxydibenzoylmethane | Neo Heliopan® 357. Parsol® 1789 | A |
| Disodium Phenyl Dibenzimidazole Tetrasulfonate | Neo Heliopan® AP | A |
| Ethylhexyl Bis-Isopentylbezoxazolylphenyl-melamine | Uvasorb® K2A | A |
| Diethylamino Hydroxybenzoyl Hexyl Benzoate | Uvinul® A Plus | A |
| Bis Ethylhexyloxyphenol Methoxyphenyl Triazine | Tinosorb® S | A/B |

(fortgesetzt)

| Sonnenschutzfilter | Markenname | Schutzbereich (UV-A/UV-B) |
|---|---|---|
| Methylene Bis-Benzotriazolyl Tetramethylbutylphenol | Tinosorb® M | A/B |
| Ethylhexyl Triazone | Uvinul® T 150 | B |
| Diethylhexyl Butamido Triazone | Uvasorb® HEB | B |
| Polysilicone-15 | Parsol® SLX | B |
| Phenylbenzimidazole Sulfonic Acid | | B |

Formulierungsbeispiel 33: O/W Sonnenschutzcreme

| Phase | Inhaltsstoff | Gew.-% |
|---|---|---|
| A | Ethylhexyl Methoxycinnamate<br>Ethylhexyltriazone<br>Benzophenone-3<br>BHT | 6,0<br>2,0<br>2,0<br>0,05 |
| B | Polymer nach Beispiel 2<br>Trilaureth-4 Phosphate<br>Polyglyceryl-2 Sesquiisostearate<br>Caprylyl Methicone<br>Phenonip®, Clariant<br>PVP/Hexadecene Copolymer<br>Tocopheryl Acetate<br>Fragrance | 1,5<br>2,0<br>1,0<br>1,0<br>0,6<br>1,0<br>0,5<br>0,2 |
| C | Wasser<br>Disodium EDTA | ad 100<br>0,1 |
| D | Methylene Bis-Benzotriazolyl Tetramethylbutylphenol | 4,0 |
| E | Mit Triethanolamine auf pH 6,8-7,2 | q.s. |

Herstellung:

Phase A homogensieren und bei 60°C lösen und in Phase B einrühren, dann Phase C unter Rühren zugeben und bei 300 Umdrehungen pro Minute rühren. Anschließend wird Phase D eingerührt und mit E der pH-Wert eingestellt.

Formulierungsbeispiel 34: Sprühbare O/W Lotion

| Phase | Inhaltsstoff | Gew.-% |
|---|---|---|
| A | Trilaureth-4 Phosphate<br>Mineral Oil<br>Isopropyl Palmitate<br>Cetearyl Alcohol<br>Caprylic/Capric Triglyceride<br>Glyceryl Stearate<br>Caprylyl Methicone | 1,0<br>8,0<br>3,0<br>0,5<br>2,0<br>0,5<br>1,0 |
| B | Polymer nach Beispiel 23 | 0,4 |
| C | Wasser<br>Glycerin | ad 100<br>5,0 |
| D | Fragrance<br>Ethanol (96 Gew.% in Wasser) | 0,3<br>5,0 |
| E | Preservative | q.s. |

Herstellung:

Phase A auf 60 C erhitzen, Phase B einrühren, dann Phase C unter Rühren zugeben und bei 300 Umdrehungen pro Minute rühren und abkühlen lassen. Phase D bei 35 °C einrühren, Phase E zugeben und schließlich homogenisieren.

Formulierungsbeispiel 35: O/W Foundation

| Phase | Inhaltsstoff | Gew.-% |
|---|---|---|
| A | Hydrogenated Polydecene<br>Caprylic/Capric Triglyceride<br>Caprylyl Trimethicone<br>Caprylyl Methicone<br>Steareth-2<br>Steareth-20<br>Polymer nach Beispiel 27 | 9,0<br>5,0<br>4,0<br>3,0<br>1,6<br>2,4<br>0,4 |
| B | Kaolin<br>Talc<br>Iron Oxide | 1,5<br>3,0<br>7,9 |
| C | Glycerin<br>Wasser | 5,0<br>ad 100 |
| D | Preservative<br>Fragrance | q.s.<br>q.s. |

Herstellung:

Phase A auf 70° C erhitzen, Phase C auf 70 °C erhitzen. Phase B in Phase A einrühren, dann Phase C zugeben und gut homogenisieren. Nach Abkühlen unter 40 °C Phase D zugeben und eine Minute homogenisieren.

## Patentansprüche

1. Wasserlösliches oder wasserquellbares Polymer enthaltend:

a) 20,0 bis 98,97 mol-% an einer oder mehreren voneinander unabhängigen wiederkehrenden Struktureinheiten der Formel (1)

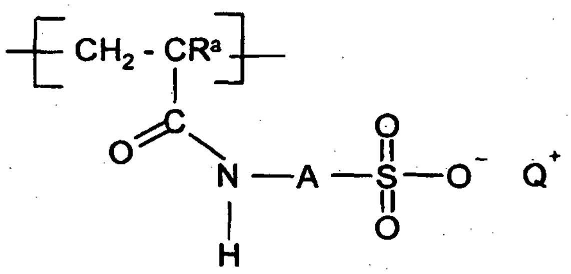

(1)

worin

$R^a$ Wasserstoff, Methyl oder Ethyl bedeutet,
A lineares oder verzweigtes $C_1$-$C_{12}$-Alkylen bedeutet, und
$Q^+$ für ein Gegenion steht

und
b) 1,0 bis 60,0 mol-% an einer oder mehreren voneinander unabhängigen wiederkehrenden Struktureinheiten der Formel (2)

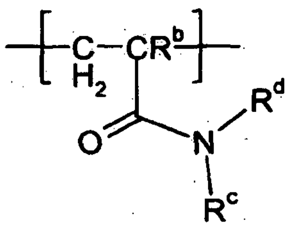

(2)

worin

$R^b$ Wasserstoff, Methyl oder Ethyl bedeutet,
$R^c$ Wasserstoff, eine lineare oder verzweigte Alkylgruppe mit 1 bis 50 Kohlenstoffatomen, eine lineare oder verzweigte Mono-Hydroxyalkylgruppe mit 2 bis 6 Kohlenstoffatomen oder eine lineare oder verzweigte Di-Hydroxyalkylgruppe mit 2 bis 6 Kohlenstoffatomen bedeutet,
$R^d$ eine lineare oder verzweigte Alkylgruppe mit 1 bis 50 Kohlenstoffatomen, eine lineare oder verzweigte Mono-Hydroxyalkylgruppe mit 2 bis 6 Kohlenstoffatomen oder eine lineare oder verzweigte Di-Hydroxyalkylgruppe mit 2 bis 6 Kohlenstoffatomen bedeutet,

und
d) 0,01 bis 8,0 mol% an einer oder mehreren voneinander unabhängigen wiederkehrenden vernetzenden Struktureinheiten der Formel (5)

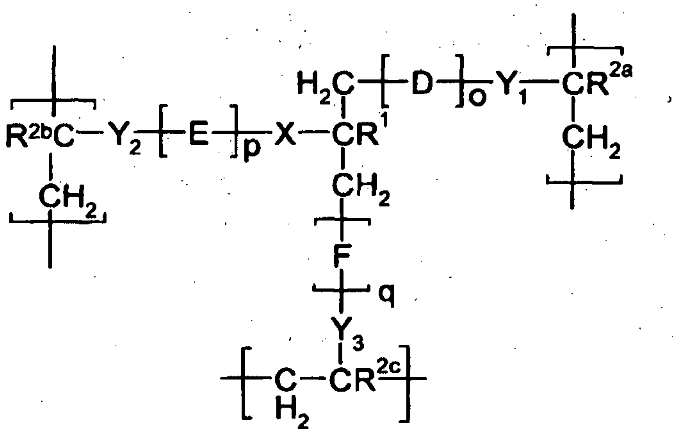

(5)

worin

$R^1$ Wasserstoff, Methyl, Ethyl, Methylol oder Ethylol bedeutet,
$R^{2a}$, $R^{2b}$ und $R^{2c}$ jeweils unabhängig voneinander Wasserstoff, Methyl oder Ethyl bedeuten,
X eine chemische Bindung, Methylen, Ethylen oder eine lineare oder verzweigte Alkylengruppe mit 3 Kohlenstoffatomen bedeutet,
$Y_1$, $Y_2$ und $Y_3$ jeweils unabhängig voneinander eine chemische Bindung, O, $CH_2$, C(O)O, OC(O), $C(O)NR^3$ oder $NR^3C(O)$ bedeuten,
$R^3$ Wasserstoff oder einen linearen oder verzweigten Alkylrest mit 1 bis 50 C-Atomen bedeutet,
D, E und F jeweils unabhängig voneinander Methylenoxy, Ethylenoxy, Propylenoxy, eine lineare oder verzweigte Alkylengruppe mit 1 bis 6 Kohlenstoffatomen, eine lineare oder verzweigte, einfach oder mehrfach ungesättigte Alkenylengruppe mit 2 bis 6 Kohlenstoffatomen, eine lineare oder verzweigte Mono-Hydroxyalkylengruppe mit 2 bis 6 Kohlenstoffatomen oder eine lineare oder verzweigte Di-Hydroxyalkylengruppe mit 3 bis 6 Kohlenstoffatomen bedeuten,
o, p und q jeweils unabhängig voneinander ganze Zahlen von 0 bis 50 bedeuten, und die Summe o + p + q $\geq$ 3 ist,

**dadurch gekennzeichnet, dass** es frei ist von Struktureinheiten der Formel (10)

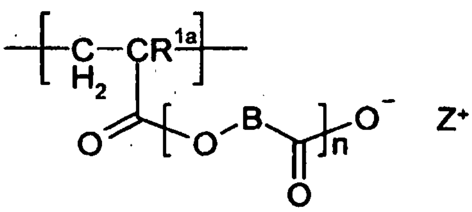

(10)

worin

$R^{1a}$ Wasserstoff, Methyl oder Ethyl bedeutet,
$Z^+$ für $H^+$, $NH_4^+$, organische Ammoniumionen $[HNR^{5b}R^{6b}R^{7b}]^+$, wobei $R^{5b}$, $R^{6b}$ und $R^{7b}$ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste $R^{5b}$, $R^{6b}$ und $R^{7b}$ nicht Wasserstoff ist, $Li^+$, $Na^+$, $K^+$, ½ $Ca^{++}$, ½ $Mg^{++}$, ½ $Zn^{++}$ oder ⅓ $Al^{+++}$ oder für Mischungen aus diesen Ionen steht,
B eine lineare oder verzweigte Alkylengruppe mit 1 bis 6 Kohlenstoffatomen ist, und
n eine ganze Zahl von 1 bis 10 ist.

2. Polymer nach Anspruch 1, **dadurch gekennzeichnet, dass** die eine oder die mehreren Struktureinheiten der Formel

(1) abgeleitet sind von der 2-Acrylamido-2-methyl-propansulfonsäure oder ihren Salzen.

3. Polymer nach Anspruch 1 oder 2, **dadurch gekennzeichnet, dass** $Q^+$ für $H^+$, $NH_4^+$, organische Ammoniumionen $[HNR^5R^6R^7]^+$, wobei $R^5$, $R^6$ und $R^7$ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine $C_6$-$C_{22}$-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste $R^5$, $R^6$ und $R^7$ nicht Wasserstoff ist, $Alkali^+$, ½ $Erdalkali^{++}$, ½ $Zn^{++}$ oder ⅓ $Al^{+++}$ oder für Mischungen aus diesen Ionen steht.

4. Polymer nach Anspruch 3, **dadurch gekennzeichnet, dass** das $Q^+$ für $H^+$, $NH_4^+$, $Alkali^+$, $Erdalkali^{++}$ oder Mischungen aus diesen Ionen steht.

5. Polymer nach einem oder mehreren der Ansprüche 1 bis 4, **dadurch gekennzeichnet, dass** der Neutralisationsgrad der einen oder der mehreren Struktureinheiten der Formel (1) von 50,0 bis 100 mol% ist.

6. Polymer nach einem oder mehreren der Ansprüche 1 bis 5, **dadurch gekennzeichnet, dass** in der einen oder den mehreren Struktureinheiten der Formel (2) $R^b$ Wasserstoff oder Methyl ist, $R^c$ H, eine lineare oder verzweigte Alkylgruppe mit 1 bis 6 Kohlenstoffatomen oder eine lineare oder verzweigte Mono-Hydroxyalkylgruppe mit 2 bis 6 Kohlenstoffatomen ist und $R^d$ eine lineare oder verzweigte Alkylgruppe mit 1 bis 6 Kohlenstoffatomen oder eine lineare oder verzweigte Mono-Hydroxyalkylgruppe mit 2 bis 6 Kohlenstoffatomen ist.

7. Polymer nach einem oder mehreren der Ansprüche 1 bis 6, **dadurch gekennzeichnet, dass** in der einen oder den mehreren Struktureinheiten der Formel (5) $R^1$ Wasserstoff oder Ethyl ist, $R^{2a}$, $R^{2b}$, und $R^{2c}$ jeweils unabhängig voneinander Wasserstoff oder Methyl sind, X eine chemische Bindung oder Methylen ist, $Y_1$, $Y_2$ und $Y_3$ jeweils unabhängig voneinander C(O)O oder OC(O) sind, D, E und F jeweils unabhängig voneinander Ethylenoxy oder Propylenoxy sind, o, p und q jeweils unabhängig voneinander ganze Zahlen von 0 bis 30 sind und die Summe o + p + q von 3 bis 20 ist.

8. Polymer nach Anspruch 7, **dadurch gekennzeichnet, dass** die eine oder die mehreren Struktureinheiten der Formel (5) hervorgegangen sind aus einem oder mehreren Vernetzern ausgewählt aus der Gruppe bestehend aus Glycerinpropoxylattriacrylat (GPTA), Trimethylolpropanpropoxytriacrylat (TMPTA-PO-3) und Trimethylolpropanethoxytriacrylat (TMPTA-EO-3).

9. Polymer nach einem oder mehreren der Ansprüche 1 bis 8, **dadurch gekennzeichnet, dass** es zusätzlich 0,01 bis 30,0 mol-%, vorzugsweise 0,1 bis 20,0 mol-% und besonders bevorzugt 0,5 bis 15,0 mol-% an einer oder mehreren voneinander unabhängigen wiederkehrenden Struktureinheiten der Formel (3)

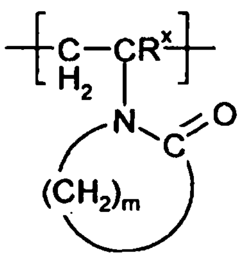

(3)

worin

$R^x$ Wasserstoff, Methyl oder Ethyl bedeutet und
m eine ganze Zahl von 2 bis 9 bedeutet,

enthält.

10. Polymer nach einem oder mehreren der Ansprüche 1 bis 9, **dadurch gekennzeichnet, dass** es zusätzlich 0,01 bis 30,0 mol-%" vorzugsweise 0,1 bis 20,0 mol-% und besonders bevorzugt 0,5 bis 15,0 mol-% an einer oder mehreren voneinander unabhängigen wiederkehrenden Struktureinheiten der Formel (4)

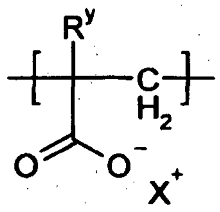

(4)

worin

$R^y$ Wasserstoff, Methyl oder Ethyl bedeutet und
$X^+$ für ein Gegenion steht,

enthält.

**11.** Polymer nach einem oder mehreren der Ansprüche 1 bis 10, **dadurch gekennzeichnet, dass** sie frei sind von Struktureinheiten der Formel (10)

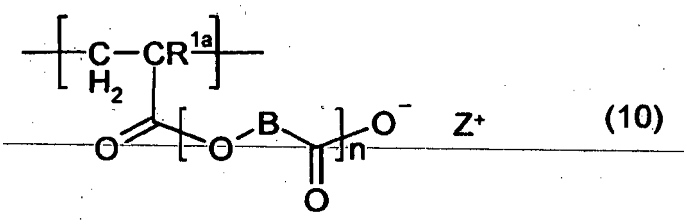

(10)

worin

$R^{1a}$ Wasserstoff, Methyl oder Ethyl bedeutet,
$Z^+$ für ein Gegenion steht,
B eine lineare oder verzweigte Alkylengruppe mit 1 bis 6 Kohlenstoffatomen ist, und
n eine ganze Zahl von 1 bis 10 ist.

**12.** Kosmetische, dermatologische oder pharmazeutische Zusammensetzung enthaltend ein oder mehrere Polymere nach einem oder mehreren der Ansprüche 1 bis 11 in einer Menge von 0,01 bis 10,0 Gew.-%, bezogen auf das Gesamtgewicht der Zusammensetzung.

**13.** Kosmetische, dermatologische oder pharmazeutische Zusammensetzung nach Anspruch 12, **dadurch gekennzeichnet, dass** sie auf wässriger oder wässrigalkoholischer Basis aufgebaut ist oder als Emulsion vorliegt.

**14.** Kosmetische, dermatologische oder pharmazeutische Zusammensetzung nach Ansprch 12 oder 13, **dadurch gekennzeichnet, dass** sie ein oder mehrere Substanzen ausgewählt aus anorganischen und organischen Salzen enthält.

**15.** Kosmetische, dermatologische oder pharmazeutische Zusammensetzung nach einem oder mehreren der Ansprüche 12 bis 14, **dadurch gekennzeichnet, dass** sie ein oder mehrere Substanzen ausgewählt aus alpha- und beta-Hydroxysäuren enthält.

**16.** Kosmetische, dermatologische oder pharmazeutische Zusammensetzung nach einem oder mehreren der Ansprüche 12 bis 15, **dadurch gekennzeichnet, dass** sie ein oder mehrere Substanzen ausgewählt aus Vitamin C und Vitamin C-Derivaten enthält.

**17.** Kosmetische, dermatologische oder pharmazeutische Zusammensetzung nach einem oder mehreren der Ansprüche 12 bis 16, **dadurch gekennzeichnet, dass** sie eine oder mehrere Substanzen ausgewählt aus Benzoesäure, Sorbinsäure, Salicylsäure, Milchsäure und Paramethoxybenzoesäure enthält.

**18.** Kosmetische, dermatologische oder pharmazeutische Zusammensetzung nach einem oder mehreren der Ansprüche 12 bis 17, **dadurch gekennzeichnet, dass** sie ein oder mehrere antimikrobielle Wirkstoffe enthält und in der Form eines Desinfektionsgels vorliegt.

**19.** Kosmetische, dermatologische oder pharmazeutische Zusammensetzung nach einem oder mehreren der Ansprüche 12 bis 18, **dadurch gekennzeichnet, dass** sie ein oder mehrere Substanzen ausgewählt aus anorganischen und organischen UV-Filtern enthält und vorzugsweise in der Form einer Sonnenschutzzusammensetzung vorliegt.

**20.** Kosmetische, dermatologische oder pharmazeutische Zusammensetzung nach einem oder mehreren der Ansprüche 12 bis 19, **dadurch gekennzeichnet, dass** sie einen pH-Wert von 2 bis 10 besitzt.

**21.** Verwendung eines oder mehrerer der Polymere nach einem oder mehreren der Ansprüche 1 bis 11 als Verdicker, Konsistenzgeber, Emulgator, Sensorikadditiv, Solubilisator, Dispergator, Gleitmittel, Haftmittel, Stabilisator oder Fließgrenzenbildner.

**22.** Verwendung eines oder mehrerer der Polymere nach Anspruch 21 zur Stabilisierung von Emulsionen und vorzugsweise zur Stabilisierung von salzhaltigen Emulsionen,

## Claims

**1.** A water-soluble or water-swellable polymer comprising:

a) 20.0 to 98.97 mol% of one or more structural units, recurring independently of one another, of the formula (1)

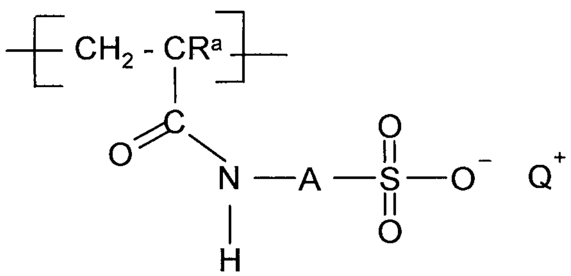

(1)

in which

$R^a$ is hydrogen, methyl or ethyl,
A is linear or branched $C_1$-$C_{12}$-alkylene, and
$Q^+$ is a counterion

and
b) 1.0 to 60.0 mol% of one or more structural units, recurring independently of one another, of the formula (2)

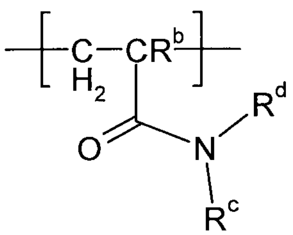

(2)

in which

$R^b$ is hydrogen, methyl or ethyl,
$R^c$ is hydrogen, a linear or branched alkyl group having 1 to 50 carbon atoms, a linear or branched mono-hydroxyalkyl group having 2 to 6 carbon atoms or a linear or branched di-hydroxyalkyl group having 2 to 6

carbon atoms,
$R^d$ is a linear or branched alkyl group having 1 to 50 carbon atoms, a linear or branched mono-hydroxyalkyl group having 2 to 6 carbon atoms or a linear or branched di-hydroxyalkyl group having 2 to 6 carbon atoms,

and
d) 0.01 to 8.0 mol% of one or more crosslinking structural units, recurring independently of one another, of the formula (5)

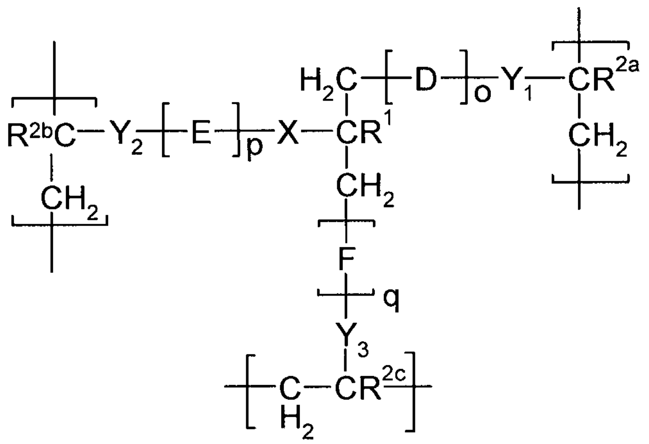

(5)

in which

$R^1$ is hydrogen, methyl, ethyl, methylol or ethylol,
$R^{2a}$ $R^{2b}$ and $R^{2c}$, in each case independently of one another, are hydrogen, methyl or ethyl,
X is a chemical bond, methylene, ethylene or a linear or branched alkylene group having 3 carbon atoms,
$Y_1$ $Y_2$ and $Y_3$, in each case independently of one another, are a chemical bond, O, $CH_2$, C(O)O, OC(O), C(O)NR or $NR^3C(O)$,
$R^3$ is hydrogen or a linear or branched alkyl radical having 1 to 50 carbon atoms,
D, E and F, in each case independently of one another, are methyleneoxy, ethyleneoxy, propyleneoxy, a linear or branched alkylene group having 1 to 6 carbon atoms, a linear or branched, mono- or polyunsaturated alkenylene group having 2 to 6 carbon atoms, a linear or branched mono-hydroxyalkylene group having 2 to 6 carbon atoms or a linear or branched di-hydroxyalkylene group having 3 to 6 carbon atoms,
o, p and q, in each case independently of one another, are integers from 0 to 50, and the sum $o + p + q$ is $\geq 3$,

wherein it is free from structural units of the formula (10)

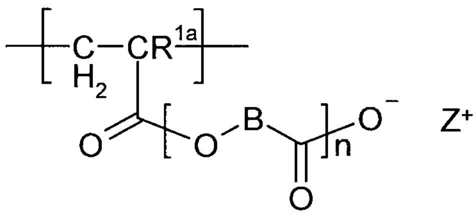

(10)

in which

$R^{1a}$ is hydrogen, methyl or ethyl,
$Z^+$ is $H^+$, $NH_4^+$, organic ammonium ions $[HNR^{5b}R^{6b}R^{7b}]^+$, where $R^{5b}$, $R^{6b}$ and $R^{7b}$, independently of one another, can be hydrogen, a linear or branched alkyl group having 1 to 22 carbon atoms, a linear or branched, mono- or polyunsaturated alkenyl group having 2 to 22 carbon atoms, a $C_6$-$C_{22}$-alkylamidopropyl group, a linear mono-hydroxyalkyl group having 2 to 10 carbon atoms or a linear or branched di-hydroxyalkyl group having 3 to 10 carbon atoms, and where at least one of the radicals $R^{5b}$, $R^{6b}$ and $R^{7b}$ is not hydrogen, , $Li^+$, $Na^+$, $K^+$, ½ $Ca^{++}$, ½ $Mg^{++}$, ½ $Zn^{++}$ or ⅓ $Al^{+++}$ or mixtures of these ions,

B is a linear or branched alkylene group having 1 to 6 carbon atoms, and
n is an integer from 1 to 10.

**2.** The polymer as claimed in claim 1, wherein the one or more structural units of the formula (1) are derived from 2-acrylamido-2-methylpropanesulfonic acid or its salts.

**3.** The polymer as claimed in claim 1 or 2, wherein $Q^+$ is $H^+$, $NH_4^+$, organic ammonium ions $[HNR^5R^6R^7]^+$, where $R^5$, $R^6$ and $R^7$, independently of one another, can be hydrogen, a linear or branched alkyl group having 1 to 22 carbon atoms, a linear or branched, mono- or polyunsaturated alkenyl group having 2 to 22 carbon atoms, a $C_6$-$C_{22}$-alkylamidopropyl group, a linear mono-hydroxyalkyl group having 2 to 10 carbon atoms or a linear or branched dihydroxyalkyl group having 3 to 10 carbon atoms, and where at least one of the radicals $R^5$, $R^6$ and $R^7$ is not hydrogen, alkali metal$^+$, ½ alkaline earth metal$^{++}$, ½ $Zn^{++}$ or ⅓ $Al^{+++}$ or mixtures of these ions.

**4.** The polymer as claimed in claim 3, wherein $Q^+$ is $H^+$, $NH_4^+$, alkali metal$^+$, alkaline earth metal$^{++}$ or mixtures of these ions.

**5.** The polymer as claimed in one or more of claims 1 to 4, wherein the degree of neutralization of the one or more structural units of the formula (1) is from 50.0 to 100 mol%.

**6.** The polymer as claimed in one or more of claims 1 to 5, wherein, in the one or more structural units of the formula (2), $R^b$ is hydrogen or methyl, $R^c$ is H, a linear or branched alkyl group having 1 to 6 carbon atoms or a linear or branched mono-hydroxyalkyl group having 2 to 6 carbon atoms and $R^d$ is a linear or branched alkyl group having 1 to 6 carbon atoms or a linear or branched mono-hydroxyalkyl group having 2 to 6 carbon atoms.

**7.** The polymer as claimed in one or more of claims 1 to 6, wherein, in the one or more structural units of the formula (5), $R^1$ is hydrogen or ethyl, $R^{2a}$, $R^{2b}$ and $R^{2c}$, in each case independently or one another are hydrogen or methyl, X is a chemical bond or methylene, $Y_1$, $Y_2$ and $Y_3$, in each case independently of one another, are C(O)O or OC(O), D, E and F, in each case independently of one another, are ethyleneoxy or propyleneoxy, o, p and q, in each case independently of one another, are integers from 0 to 30 and the sum o + p + q is from 3 to 20.

**8.** The polymer as claimed in claim 7, wherein the one or more structural units of the formula (5) are derived from one or more crosslinkers selected from the group consisting of glycerol propoxylate triacrylate (GPTA), trimethylolpropane propoxy triacrylate (TMPTA-PO-3) and trimethylolpropane ethoxy triacrylate (TMPTA-EO-3).

**9.** The polymer as claimed in one or more of claims 1 to 8, wherein it additionally comprises 0.01 to 30.0 mol%, preferably 0.1 to 20.0 mol% and particularly preferably 0.5 to 15.0 mol% of one or more structural units, recurring independently of one another, of the formula (3)

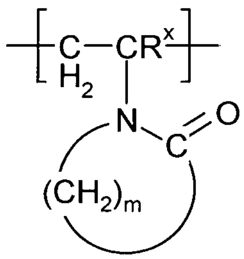

(3)

in which

$R^x$ is hydrogen, methyl or ethyl and
m is an integer from 2 to 9.

**10.** The polymer as claimed in one or more of claims 1 to 9, wherein it additionally comprises 0.01 to 30.0 mol%, preferably 0.1 to 20.0 mol% and particularly preferably 0.5 to 15.0 mol%, of one or more structural units, recurring independently of one another, of the formula (4)

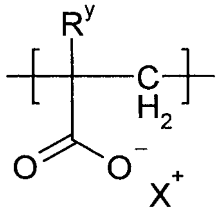

(4)

in which

$R^y$ is hydrogen, methyl or ethyl and
$X^+$ is a counterion.

**11.** The polymer as claimed in one or more of claims 1 to 10, wherein it is free from structural units of the formula (10)

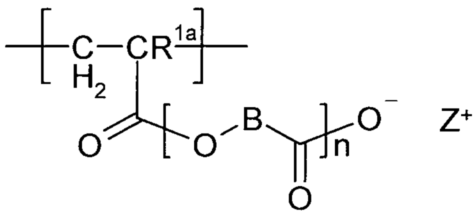

(10)

in which

$R^{1a}$ is hydrogen, methyl or ethyl,
$Z^+$ is a counterion,
B is a linear or branched alkylene group having 1 to 6 carbon atoms, and
n is an integer from 1 to 10.

**12.** A cosmetic, dermatological or pharmaceutical composition comprising one or more polymers as claimed in one or more of claims 1 to 11 in an amount of from 0.01 to 10.0% by weight, based on the total weight of the composition.

**13.** The cosmetic, dermatological or pharmaceutical composition as claimed in claim 12, wherein it is formulated on an aqueous or aqueous-alcoholic basis or is present in the form of an emulsion.

**14.** The cosmetic, dermatological or pharmaceutical composition as claimed in claim 12 or 13, wherein it comprises one or more substances selected from inorganic and organic salts.

**15.** The cosmetic, dermatological or pharmaceutical composition as claimed in one or more of claims 12 to 14, wherein it comprises one or more substances selected from alpha- and beta-hydroxy acids.

**16.** The cosmetic, dermatological or pharmaceutical composition as claimed in one or more of claims 12 to 15, wherein it comprises one or more substances selected from vitamin C and vitamin C derivatives.

**17.** The cosmetic, dermatological or pharmaceutical composition as claimed in one or more of claims 12 to 16, wherein it comprises one or more substances selected from benzoic acid, sorbic acid, salicylic acid, lactic acid and paramethoxybenzoic acid.

**18.** The cosmetic, dermatological or pharmaceutical composition as claimed in one or more of claims 12 to 17, wherein it comprises one or more antimicrobial active ingredients and is present in the form of a disinfection gel.

**19.** The cosmetic, dermatological or pharmaceutical composition as claimed in one or more of claims 12 to 18, wherein it comprises one or more substances selected from inorganic and organic UV filters and is preferably present in the form of a sunscreen composition.

**20.** The cosmetic, dermatological or pharmaceutical composition as claimed in one or more of claims 12 to 19, wherein it has a pH of from 2 to 10.

**21.** The use of one or more of the polymers according to one or more of claims 1 to 11 as thickener, consistency regulator, emulsifier, sensory additive, solubilizer, dispersant, lubricant, adhesive, stabilizer or yield point former.

**22.** The use of one or more of the polymers as claimed in claim 21 for stabilizing emulsions and preferably for stabilizing salt-containing emulsions.

## Revendications

**1.** Polymère soluble dans l'eau ou apte à gonfler dans l'eau, contenant :

a) 20,0 à 98,97 % en moles d'un ou de plusieurs motifs structuraux répétitifs indépendants les uns des autres, de formule (1)

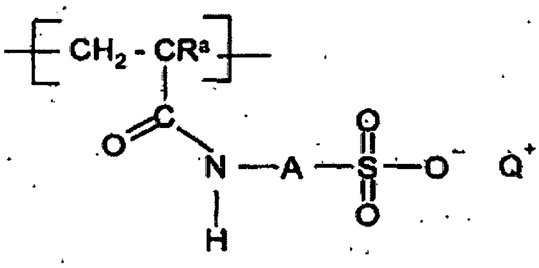

(1)

dans laquelle

$R^a$ représente un atome d'hydrogène, le groupe méthyle ou éthyle,
A représente un groupe alkylène en $C_1$-$C_{12}$ linéaire ou ramifié, et
$Q^+$ représente un ion opposé

et
b) 1,0 à 60,0 % en moles d'un ou de plusieurs motifs structuraux répétitifs indépendants les uns des autres, de formule (2)

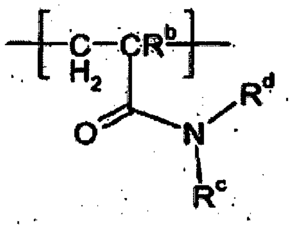

(2)

dans laquelle

$R^b$ représente un atome d'hydrogène, le groupe méthyle ou éthyle,
$R^c$ représente un atome d'hydrogène, un groupe alkyle linéaire ou ramifié ayant de 1 à 50 atomes de carbone, un groupe monohydroxyalkyle linéaire ou ramifié ayant de 2 à 6 atomes de carbone ou un groupe di-hydroxyalkyle linéaire ou ramifié ayant de 2 à 6 atomes de carbone,
$R^d$ représente un groupe alkyle linéaire ou ramifié ayant de 1 à 50 atomes de carbone, un groupe mono-hydroxyalkyle linéaire ou ramifié ayant de 2 à 6 atomes de carbone ou un groupe di-hydroxyalkyle linéaire ou ramifié ayant de 2 à 6 atomes de carbone,

et

d) 0,01 à 8,0 % en moles d'un ou de plusieurs motifs structuraux réticulants répétitifs, indépendants les uns des autres, de formule (5)

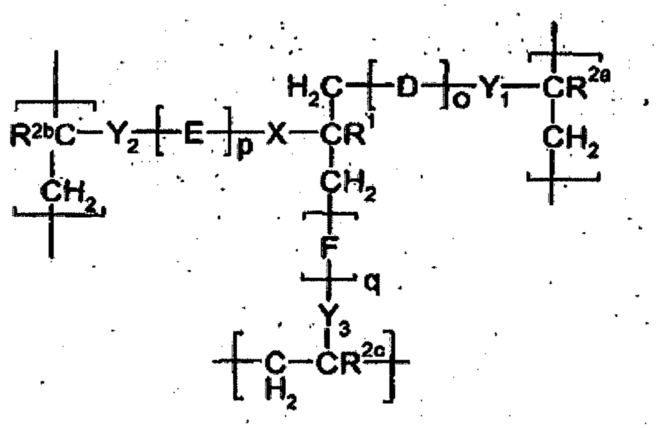

(5)

dans laquelle

$R^1$ représente un atome d'hydrogène, le groupe méthyle, éthyle, méthylol ou éthylol,
$R^{2a}$, $R^{2b}$ et $R^{2c}$ représentent chacun indépendamment un atome d'hydrogène, le groupe méthyle ou éthyle,
X représente une liaison chimique, le groupe méthylène, éthylène ou un groupe alkylène linéaire ou ramifié ayant 3 atomes de carbone,
$Y_1$, $Y_2$ et $Y_3$ représentent chacun indépendamment une liaison chimique, O, $CH_2$, C(O)O, OC(O), $C(O)NR^3$ ou $NR^3C(O)$,
$R^3$ représente un atome d'hydrogène ou un radical alkyle linéaire ou ramifié ayant de 1 à 50 atomes de carbone,
D, E et F représentent chacun indépendamment le groupe méthylène-oxy, éthylène-oxy, propylène-oxy, un groupe alkylène linéaire ou ramifié ayant de 1 à 6 atomes de carbone, un groupe alcénylène linéaire ou ramifié, une ou plusieurs fois insaturé, ayant de 2 à 6 atomes de carbone, un groupe mono-hydroxyalkylène linéaire ou ramifié ayant de 2 à 6 atomes de carbone ou un groupe di-hydroxyalkylène linéaire ou ramifié ayant de 3 à 6 atomes de carbone,
o, p et q représentent chacun indépendamment des nombres entiers valant de 0 à 50, et la somme o + p + q est $\geq 3$,

**caractérisé en ce qu'**il est exempt de motifs structuraux de formule (10)

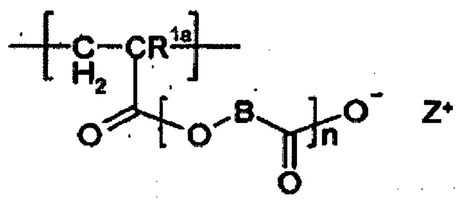

(10)

dans laquelle

$R^{1a}$ représente un atome d'hydrogène, le groupe méthyle ou éthyle,
$Z^+$ représente $H^+$, $NH_4^+$, des ions ammonium organiques $[HNR^{5b}R^{6b}R^{7b}]^+$, $R^{5b}$, $R^{6b}$ et $R^{7b}$ pouvant être chacun indépendamment un atome d'hydrogène, un groupe alkyle linéaire ou ramifié ayant de 1 à 22 atomes de carbone, un groupe alcényle une ou plusieurs fois insaturé, linéaire ou ramifié, ayant de 2 à 22 atomes de carbone, un groupe alkyl ($C_6$-$C_{22}$) - amidopropyle, un groupe mono-hydroxyalkyle linéaire ayant de 2 à 10 atomes de carbone ou un groupe di-hydroxyalkyle linéaire ou ramifié ayant de 3 à 10 atomes de carbone, et au moins l'un des radicaux $R^{5b}$, $R^{6b}$ et $R^{7b}$ n'étant pas un atome d'hydrogène, représente $Li^+$,

$Na^+$, $K^+$, ½ $Ca^{++}$, ½ $Mg^{++}$, ½ $Zn^{++}$ ou $\frac{1}{3}$ $Al^{+++}$ ou des mélanges de ces ions,
B est un groupe alkylène linéaire ou ramifié ayant de 1 à 6 atomes de carbone, et
n est un nombre entier valant de 1 à 10.

2. Polymère selon la revendication 1, **caractérisé en ce que** ledit un ou lesdits plusieurs motifs structuraux de formule (1) sont dérivés de l'acide 2-acrylamido-2-méthyl-propanesulfonique ou de ses sels.

3. Polymère selon la revendication 1 ou 2, **caractérisé en ce que** $Q^+$ représente $H^+$, $NH_4^+$, des ions ammonium organiques $[NHR^5R^6R^7]^+$, $R^5$, $R^6$ et $R^7$ représentant chacun indépendamment un atome d'hydrogène, un groupe alkyle linéaire ou ramifié ayant de 1 à 22 atomes de carbone, un groupe alcényle une ou plusieurs fois insaturé, linéaire ou ramifié, ayant de 2 à 22 atomes de carbone, un groupe alkyl($C_6$-$C_{22}$)amidopropyle, un groupe mono-hydroxyalkyle linéaire ayant de 2 à 10 atomes de carbone ou un groupe di-hydroxyalkyle linéaire ou ramifié ayant de 3 à 10 atomes de carbone, et au moins l'un des radicaux $R^5$, $R^6$ et $R^7$ n'étant pas un atome d'hydrogène, représente un ion de métal alcalin$^+$, un ½ ion de métal alcalino-terreux$^{++}$, ½ $Zn^{++}$ ou $\frac{1}{3}$ $Al^{+++}$ ou des mélanges de ces ions.

4. Polymère selon la revendication 3, **caractérisé en ce que** $Q^+$ représente $H^+$, $NH_4^+$, un ion de métal alcalin$^+$, un ion de métal alcalino-terreux$^{++}$ ou des mélanges de ces ions.

5. Polymère selon une ou plusieurs des revendications 1 à 4, **caractérisé en ce que** le degré de neutralisation dudit un ou desdits plusieurs motifs structuraux de formule (1) vaut de 50,0 à 100 % en moles.

6. Polymère selon une ou plusieurs des revendications 1 à 5, **caractérisé en ce que** dans ledit un ou lesdits plusieurs motifs structuraux de formule (2) $R^b$ représente un atome d'hydrogène ou le groupe méthyle, $R^6$ est H, un groupe alkyle linéaire ou ramifié ayant de 1 à 6 atomes de carbone ou un groupe mono-hydroxyalkyle linéaire ou ramifié ayant de 2 à 6 atomes de carbone et $R^d$ est un groupe alkyle linéaire ou ramifié ayant de 1 à 6 atomes de carbone ou un groupe mono-hydroxyalkyle linéaire ou ramifié ayant de 2 à 6 atomes de carbone.

7. Polymère selon une ou plusieurs des revendications 1 à 6, **caractérisé en ce que** dans ledit un ou lesdits plusieurs motifs structuraux de formule (5) $R^1$ est un atome d'hydrogène ou le groupe éthyle, $R^{2a}$, $R^{2b}$ et $R^{2c}$ représentent chacun indépendamment un atome d'hydrogène ou le groupe méthyle, X est une liaison chimique ou le groupe méthylène, $Y_1$, $Y_2$ et $Y_3$ représentent chacun indépendamment C(O)O ou OC(O), D, E, F représentent chacun indépendamment le groupe éthylène-oxy ou propylène-oxy, o, p et q sont chacun indépendamment des nombres entiers valant de 0 à 30 et la somme o + p + q vaut de 3 à 20.

8. Polymère selon la revendication 7, **caractérisé en ce que** ledit un ou lesdits plusieurs motifs structuraux de formule (5) sont issus d'un ou de plusieurs agents de réticulation choisis dans le groupe constitué par le propoxylate-triacrylate de glycérol (GPTA), le propoxytriacrylate de triméthylolpropane (TMPTA-PO-3) et l'éthoxytriacrylate de triméthylolpropane (TMPTA-EO-3).

9. Polymère selon une ou plusieurs des revendications 1 à 8, **caractérisé en ce qu'**il contient en outre 0,01 à 30,0 % en moles, de préférence 0,1 à 20,0 % en moles et de façon particulièrement préférée 0,5 à 15,0 % en moles d'un ou de plusieurs motifs structuraux indépendants les uns des autres, de formule (3)

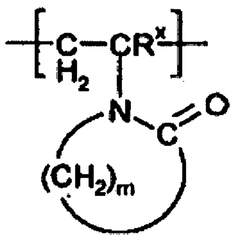

(3)

dans laquelle

$R^x$ représente un atome d'hydrogène, le groupe méthyle ou éthyle et
m représente un nombre entier valant de 2 à 9.

**10.** Polymère selon une ou plusieurs des revendications 1 à 9, **caractérisé en ce qu'**il contient en outre 0,01 à 30,0 % en moles, de préférence 0,1 à 20,0 % en moles et de façon particulièrement préférée 0,5 à 15,0 % en moles d'un ou de plusieurs motifs structuraux répétitifs indépendants les uns des autres, de formule (4)

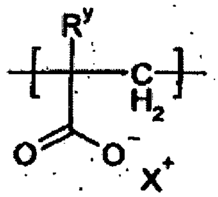

(4)

dans laquelle

$R^y$ représente un atome d'hydrogène, le groupe méthyle ou éthyle et
$X^+$ représente un ion opposé.

**11.** Polymère selon une ou plusieurs des revendications 1 à 10, **caractérisé en ce qu'**il est exempt de motifs structuraux de formule (10)

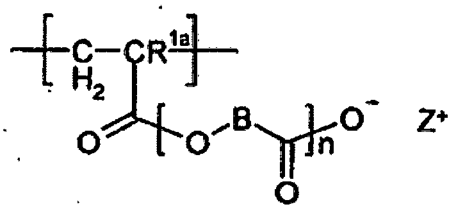

(10)

dans laquelle

$R^{1a}$ représente un atome d'hydrogène, le groupe méthyle ou éthyle,
$Z^+$ représente un ion opposé,
B est un groupe alkylène linéaire ou ramifié ayant de 1 à 6 atomes de carbone, et
n est un nombre entier valant de 1 à 10.

**12.** Composition cosmétique, dermatologique ou pharmaceutique contenant un ou plusieurs polymères selon une ou plusieurs des revendications 1 à 11 en une quantité de 0,01 à 10,0 % en poids, par rapport au poids total de la composition.

**13.** Composition cosmétique, dermatologique ou pharmaceutique selon la revendication 12, **caractérisée en ce qu'**elle est constituée sur une base aqueuse ou aqueuse-alcoolique ou se trouve sous forme d'émulsion.

**14.** Composition cosmétique, dermatologique ou pharmaceutique selon la revendication 12 ou 13, **caractérisée en ce qu'**elle contient une ou plusieurs substances choisies parmi des sels inorganiques et des sels organiques.

**15.** Composition cosmétique, dermatologique ou pharmaceutique selon une ou plusieurs des revendications 12 à 14, **caractérisée en ce qu'**elle contient une ou plusieurs substances choisies parmi des acides alpha- et bêta-hydroxylés.

**16.** Composition cosmétique, dermatologique ou pharmaceutique selon une ou plusieurs des revendications 12 à 15, **caractérisée en ce qu'**elle contient une ou plusieurs substances choisies parmi la vitamine C et des dérivés de vitamine C.

**17.** Composition cosmétique, dermatologique ou pharmaceutique selon une ou plusieurs des revendications 12 à 16,

**caractérisée en ce qu'**elle contient une ou plusieurs substances choisies parmi l'acide benzoïque, l'acide sorbique, l'acide salicylique, l'acide lactique et l'acide paraméthoxybenzoïque.

**18.** Composition cosmétique, dermatologique ou pharmaceutique selon une ou plusieurs des revendications 12 à 17, **caractérisée en ce qu'**elle contient une ou plusieurs substances actives antimicrobiennes et se trouve sous la forme d'un gel désinfectant.

**19.** Composition cosmétique, dermatologique ou pharmaceutique selon une ou plusieurs des revendications 12 à 18, **caractérisée en ce qu'**elle contient une ou plusieurs substances choisies parmi des filtres UV organiques et des filtres UV inorganiques et se trouve de préférence sous la forme d'une composition antisolaire.

**20.** Composition cosmétique, dermatologique ou pharmaceutique selon une ou plusieurs des revendications 12 à 19, **caractérisée en ce qu'**elle présente un pH de 2 à 10.

**21.** Utilisation d'un ou de plusieurs des polymères selon une ou plusieurs des revendications 1 à 11 en tant qu'épaississant, agent de consistance, émulsifiant, lubrifiant, additif sensoriel, solubilisant, dispersant, adhésif, stabilisant ou générateur de limite d'écoulement.

**22.** Utilisation d'un ou de plusieurs polymères selon la revendication 21 pour la stabilisation d'émulsions et de préférence pour la stabilisation d'émulsions contenant des sels.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0816403 A, Clariant
- EP 0815843 A
- EP 0815844 A
- EP 0815845 A
- EP 0815846 A
- EP 0815847 A
- EP 0815828 A
- EP 0829258 A
- EP 1136058 A
- EP 1325729 A
- EP 1468670 A
- WO 2010108634 A
- EP 1746114 A, Shiseido
- WO 2010009953 A, Henkel
- EP 1116733 A, Clariant
- JP 2005206607 A, Shiseido


### In der Beschreibung aufgeführte Nicht-Patentliteratur


- **BERND TIEKE.** Makromolekulare Chemie: Eine Einführung. Wiley-VCH, 09. September 2005